# EUROPEAN PATENT APPLICATION

(11) **EP 4 173 480 A1**
(43) Date of publication of application: **03.05.2023**
(21) Application number: 20942217.9
(22) Date of filing: 25.06.2020
(51) Int. Cl.: A01K 67/027

(54) **HETEROZYGOUS TRANSGENIC ANIMAL**

(71) Applicant: HUMAB CO. LTD., Seoul 07793 (KR)
(72) Inventor: OH, Chang Kyu, Seoul 03012 (KR); OH, Kyoung Seok, Seoul 03471 (KR); PARK, Soon Ik, Seoul 04385 (KR)
(74) Representative: dompatent von Kreisler Selting Werner - Partnerschaft von Patent- und Rechtsanwälten mbB
(86) International application number: PCT/KR2020/008268
(87) International publication number: WO 2021/261620

(57) **Abstract**

The present invention relates to a transgenic animal having a genome including a humanized immunoglobulin locus for securing the diversity of an antibody repertoire and a method of producing the same. More specifically, the present invention relates to a transgenic non-human animal having two alleles of a humanized immunoglobulin gene and a method of producing the same, wherein the two alleles are hetero-alleles.

## Description

### TECHNICAL FIELD

The present invention relates to a transgenic animal having a genome including a humanized immunoglobulin locus for securing the diversity of an antibody repertoire and a method of producing the same. More specifically, the present invention relates to a transgenic non-human animal having two alleles of a humanized immunoglobulin gene and a method of producing the same, wherein the two alleles are hetero-alleles.

### BACKGROUND ART

One of the response mechanisms that occur when our body is exposed to external pathogens is the production of an antibody specific to the corresponding pathogen. Antibodies are proteins produced by B cells, and it is essential to secure various antibodies to protect the body from numerous pathogens exposed throughout its lifetime. As mechanisms for securing diversity, there are VDJ recombination, somatic hypermutation (SHM), and class switching. When V, D, and J segments are recombined at the DNA level one by one through the mechanism, somatic mutation occurs by activation-induced cytidine (AID) deaminase. The variable region of the antibody thus determined is recombined with the constant region of the immunoglobulin M, D, A, G or E class. The antibody repertoire, which is a total of such various antibodies, theoretically has a diversity of 10⁹ or more. When our body is exposed to a pathogen, in the antibody repertoire a very small fraction of B cells with high specificity for the pathogen that is an antigen, , is amplified to produce antibodies, thereby defending the body from the pathogen. Thus, an increase in the diversity of the antibody repertoire enables the expansion of a candidate group of antigen-specific B cells (or antibodies) and, at the same time, enables the production and selection of B cells (or antibodies) with high specificity, and thus efforts are needed to increase antibody repertoire diversity in the development of antibody-producing transgenic animals.

### DESCRIPTION OF EMBODIMENTS

### TECHNICAL PROBLEM

An object of the present invention is to provide a transgenic animal having a genome including a humanized immunoglobulin locus.

Another object of the present invention is to provide a method of producing a transgenic animal having a genome including a humanized immunoglobulin locus.

### TECHNICAL SOLUTION

According to an aspect of the present invention, there is provided a transgenic animal having a genome including a humanized immunoglobulin locus.

The transgenic animal having a genome including a humanized immunoglobulin locus may be a transgenic non-human animal having two alleles of a humanized immunoglobulin gene. In this case, the two alleles of a humanized immunoglobulin gene may be different, and thus, the transgenic non-human animal may be heterozygous at the humanized immunoglobulin locus.

The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

The humanized immunoglobulin gene may include a partial region of a human immunoglobulin gene and a partial region of a non-human animal immunoglobulin gene. Alternatively, the humanized immunoglobulin gene may include a human immunoglobulin gene instead of a non-human animal immunoglobulin gene.

In this case, the partial region of the human immunoglobulin gene may be a variable region of the human immunoglobulin gene. The variable region may be a non-rearrranged variable region. In this case, the unrearranged variable region may include V, D, and J segments, or V and J segments.

In this case, the partial region of the non-human animal immunoglobulin gene may be a constant region of the non-human animal immunoglobulin gene (endogenous immunoglobulin gene). The constant region may include a C segment.

The two alleles of the humanized immunoglobulin gene may be hetero-alleles.

In this case, the hetero-alleles may be a first humanized immunoglobulin gene and a second humanized immunoglobulin gene.

In this case, the first humanized immunoglobulin gene may include a first unrearranged variable region of a first human immunoglobulin gene. The second humanized immunoglobulin gene may include a second unrearranged variable region of a second human immunoglobulin gene.

In this case, the first unrearranged variable region and the second unrearranged variable region may be hetero variable regions.

In this case, the first unrearranged variable region and the second unrearranged variable region may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

In this case, the first human immunoglobulin gene may be derived from a first human individual, and the second human immunoglobulin gene may be derived from a second human individual. In this case, the first human individual and the second human individual may be different individuals.

In this case, the first human immunoglobulin gene may be derived from an individual belonging to a first population(race), and the second human immunoglobulin gene may be derived from an individual belonging to a second population(race). In this case, the first population(race) and the second population(race) may be different. In this case, each of the first population(race) and the second population(race) may be a population(race) selected from the group consisting of Caucasian (the Caucasoid race), Mongolian (the Mongoloid race), Negro (Negroid), the Malay race, Polynesian, and the Australoid race.

The transgenic animal having a genome including a humanized immunoglobulin locus may be a transgenic non-human animal having alleles of an immunoglobulin, the alleles being derived from two different races.

In one embodiment, the transgenic animal may be a transgenic non-human animal having a genome including humanized immunoglobulin heavy chain gene loci (humanized IGH loci). The transgenic non-human animal may be a transgenic non-human animal having two alleles of a humanized immunoglobulin heavy chain gene (humanized IGH). The humanized immunoglobulin heavy chain gene may include a variable region of a human immunoglobulin heavy chain gene and a constant region of a non-human animal immunoglobulin heavy chain gene. In this case, the variable region of a human immunoglobulin heavy chain gene may include unrearranged V, D, and J segments. The two alleles of a humanized immunoglobulin heavy chain gene may be hetero-alleles. The hetero-alleles may be a first humanized immunoglobulin heavy chain gene and a second humanized immunoglobulin heavy chain gene. In this case, the first humanized immunoglobulin heavy chain gene may include a first unrearranged variable region of a first human immunoglobulin heavy chain gene. The second humanized immunoglobulin heavy chain gene may include a second unrearranged variable region of a second human immunoglobulin heavy chain gene. In this case, the first unrearranged variable region and the second unrearranged variable region may be hetero-variable regions. Herein, the hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the first human immunoglobulin heavy chain gene may be derived from a first human individual, and the second human immunoglobulin heavy chain gene may be derived from a second human individual. The first human individual and the second human individual may be different. Alternatively, the first human immunoglobulin heavy chain gene may be derived from an individual belonging to a first population(race), and the second human immunoglobulin heavy chain gene may be derived from an individual belonging to a second population(race). The first population(race) and the second population(race) may be different. In this case, each of the first population(race) and the second population(race) may be a population(race) selected from the group consisting of Caucasian (the Caucasoid race), Mongolian (the Mongoloid race), Negro (Negroid), the Malay race, Polynesian, and the Australoid race. For example, the first race may be Caucasian, and the second race may be Negro. Thus, the transgenic non-human animal may be a transgenic non-human animal having two alleles derived from different races for an immunoglobulin heavy chain gene. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In another embodiment, the transgenic animal may be a transgenic non-human animal having a genome including humanized immunoglobulin kappa gene loci (humanized IGK loci). The transgenic non-human animal may be a transgenic non-human animal having two alleles of a humanized immunoglobulin kappa gene (humanized IGK). The humanized immunoglobulin kappa gene may include a variable region of a human immunoglobulin kappa gene and a constant region of a non-human animal immunoglobulin kappa gene. In this case, the variable region of a human immunoglobulin kappa gene may include unrearranged V and J segments. The two alleles of a humanized immunoglobulin kappa gene may be hetero-alleles. The hetero-alleles may be a first humanized immunoglobulin kappa gene and a second humanized immunoglobulin kappa gene. In this case, the first humanized immunoglobulin kappa gene may include a first unrearranged variable region of a first human immunoglobulin kappa gene. The second humanized immunoglobulin kappa gene may include a second unrearranged variable region of a second human immunoglobulin kappa gene. In this case, the first unrearranged variable region and the second unrearranged variable region may be hetero-variable regions. In this case, the hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the first human immunoglobulin kappa gene may be derived from a first human individual, and the second human immunoglobulin kappa gene may be derived from a second human individual. The first human individual and the second human individual may be different. Alternatively, the first human immunoglobulin kappa gene may be derived from an individual belonging to a first population(race), and the second human immunoglobulin kappa gene may be derived from an individual belonging to a second population(race). The first population(race) and the second population(race) may be different. In this case, each of the first population(race) and the second population(race) may be a population(race) selected from the group consisting of Caucasian (the Caucasoid race), Mongolian (the Mongoloid race), Negro (Negroid), the Malay race, Polynesian, and the Australoid race. For example, the first race may be Caucasian, and the second race may be Mongolian. Thus, the transgenic non-human animal may be a transgenic non-human animal having two alleles derived from different races for an immunoglobulin kappa gene. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In another embodiment, the transgenic animal may be a transgenic non-human animal having a genome including humanized immunoglobulin lambda gene loci (humanized IGL loci). The transgenic non-human animal may be a transgenic non-human animal having two alleles of a humanized immunoglobulin lambda gene (humanized IGL). The humanized immunoglobulin lambda gene may include a variable region of a human immunoglobulin lambda gene and a constant region of a non-human animal immunoglobulin lambda gene. In this case, the variable region of a human immunoglobulin lambda gene may include unrearranged V and J segments. The two alleles of a humanized immunoglobulin lambda gene may be hetero-alleles. The hetero-alleles may be a first humanized immunoglobulin lambda gene and a second humanized immunoglobulin lambda gene. In this case, the first humanized immunoglobulin lambda gene may include a first unrearranged variable region of a first human immunoglobulin lambda gene. The second humanized immunoglobulin lambda gene may include a second unrearranged variable region of a second human immunoglobulin lambda gene. In this case, the first unrearranged variable region and the second unrearranged variable region may be hetero-variable regions. In this case, the hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the first human immunoglobulin lambda gene may be derived from a first human individual, and the second human immunoglobulin lambda gene may be derived from a second human individual. The first human individual and the second human individual may be different. Alternatively, the first human immunoglobulin lambda gene may be derived from an individual belonging to a first population(race), and the second human immunoglobulin lambda gene may be derived from an individual belonging to a second population(race). The first population(race) and the second population(race) may be different. In this case, each of the first population(race) and the second population(race) may be a population(race) selected from the group consisting of Caucasian (the Caucasoid race), Mongolian (the Mongoloid race), Negro (Negroid), the Malay race, Polynesian, and the Australoid race. For example, the first race may be Negro, and the second race may be a Mongolian race. Thus, the transgenic non-human animal may be a transgenic non-human animal having two alleles derived from different races for an immunoglobulin lambda gene. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In one embodiment, the transgenic animal may be a transgenic non-human animal having a genome including humanized immunoglobulin heavy chain gene loci (humanized IGH loci) and humanized immunoglobulin kappa gene loci (humanized IGK loci). The transgenic non-human animal may be a transgenic non-human animal having two alleles of each of a humanized immunoglobulin heavy chain gene (humanized IGH) and a humanized immunoglobulin kappa gene (humanized IGK). The humanized immunoglobulin heavy chain gene may include a variable region of a human immunoglobulin heavy chain gene and a constant region of a non-human animal immunoglobulin heavy chain gene. In this case, the variable region of a human immunoglobulin heavy chain gene may include unrearranged V, D, and J segments. The two alleles of the humanized immunoglobulin heavy chain gene may be hetero-alleles. The hetero-alleles of the humanized immunoglobulin heavy chain gene may be a first humanized immunoglobulin heavy chain gene and a second humanized immunoglobulin heavy chain gene. In this case, the first humanized immunoglobulin heavy chain gene may include a first unrearranged variable region of a first human immunoglobulin heavy chain gene. The second humanized immunoglobulin heavy chain gene may include a second unrearranged variable region of a second human immunoglobulin heavy chain gene. In this case, the first unrearranged variable region and the second unrearranged variable region may be heavy chain hetero-variable regions. In this case, the heavy chain hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the first human immunoglobulin heavy chain gene may be derived from a first human individual, and the second human immunoglobulin heavy chain gene may be derived from a second human individual. The first human individual and the second human individual may be different. Alternatively, the first human immunoglobulin heavy chain gene may be derived from an individual belonging to a first population(race), and the second human immunoglobulin heavy chain gene may be derived from an individual belonging to a second population(race). The first population(race) and the second population(race) may be different. The humanized immunoglobulin kappa gene may include a variable region of a human immunoglobulin kappa gene and a constant region of a mouse immunoglobulin kappa gene. In this case, the variable region of a human immunoglobulin kappa gene may include unrearranged V and J segments. The two alleles of the humanized immunoglobulin kappa gene may be hetero-alleles. The hetero-alleles of the humanized immunoglobulin kappa gene may be a third humanized immunoglobulin kappa gene and a fourth humanized immunoglobulin kappa gene. In this case, the third humanized immunoglobulin kappa gene may include a third unrearranged variable region of a third human immunoglobulin kappa gene. The fourth humanized immunoglobulin kappa gene may include a fourth unrearranged variable region of a fourth human immunoglobulin kappa gene. In this case, the third unrearranged variable region and the fourth unrearranged variable region may be kappa hetero-variable regions. In this case, the kappa hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the third human immunoglobulin kappa gene may be derived from a third human individual, and the fourth human immunoglobulin kappa gene may be derived from a fourth human individual. The third human individual and the fourth human individual may be different. Alternatively, the third human immunoglobulin kappa gene may be derived from an individual belonging to a third population(race), and the fourth human immunoglobulin kappa gene may be derived from an individual belonging to a fourth population(race). The third population(race) and the fourth population(race) may be different. In this case, the third population(race) may be the same as the first population(race). Alternatively, the first population(race), the second population(race), the third population(race), and the fourth population(race) may be different. In this case, each of the first population(race), the second population(race), the third population(race), and the fourth population(race) may be a population(race) selected from the group consisting of Caucasian (the Caucasoid race), Mongolian (the Mongoloid race), Negro (Negroid), the Malay race, Polynesian, and the Australoid race. Thus, the transgenic non-human animal may be a transgenic non-human animal having two alleles derived from different races for an immunoglobulin heavy chain gene and two alleles derived from different races for an immunoglobulin kappa gene. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In another embodiment, the transgenic animal may be a transgenic non-human animal having a genome including humanized immunoglobulin heavy chain gene loci (humanized IGH loci) and humanized immunoglobulin lambda gene loci (humanized IGL loci). The transgenic non-human animal may be a transgenic non-human animal having two alleles of each of a humanized immunoglobulin heavy chain gene (humanized IGH) and a humanized immunoglobulin lambda gene (humanized IGL). The humanized immunoglobulin heavy chain gene may include a variable region of a human immunoglobulin heavy chain gene and a constant region of a non-human animal immunoglobulin heavy chain gene. In this case, the variable region of a human immunoglobulin heavy chain gene may include unrearranged V, D, and J segments. The two alleles of the humanized immunoglobulin heavy chain gene may be hetero-alleles. The hetero-alleles of the humanized immunoglobulin heavy chain gene may be a first humanized immunoglobulin heavy chain gene and a second humanized immunoglobulin heavy chain gene. In this case, the first humanized immunoglobulin heavy chain gene may include a first unrearranged variable region of a first human immunoglobulin heavy chain gene. The second humanized immunoglobulin heavy chain gene may include a second unrearranged variable region of a second human immunoglobulin heavy chain gene. In this case, the first unrearranged variable region and the second unrearranged variable region may be heavy chain hetero-variable regions. In this case, the heavy chain hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the first human immunoglobulin heavy chain gene may be derived from a first human individual, and the second human immunoglobulin heavy chain gene may be derived from a second human individual. The first human individual and the second human individual may be different. Alternatively, the first human immunoglobulin heavy chain gene may be derived from an individual belonging to a first population(race), and the second human immunoglobulin heavy chain gene may be derived from an individual belonging to a second population(race). The first population(race) and the second population(race) may be different. The humanized immunoglobulin lambda gene may include a variable region of a human immunoglobulin lambda gene and a constant region of a non-human animal immunoglobulin lambda gene. In this case, the variable region of a human immunoglobulin lambda gene may include unrearranged V and J segments. The two alleles of the humanized immunoglobulin lambda gene may be hetero-alleles. The hetero-alleles of the humanized immunoglobulin lambda gene may be a third humanized immunoglobulin lambda gene and a fourth humanized immunoglobulin lambda gene. In this case, the third humanized immunoglobulin lambda gene may include a third unrearranged variable region of a third human immunoglobulin lambda gene. The fourth humanized immunoglobulin lambda gene may include a fourth unrearranged variable region of a fourth human immunoglobulin lambda gene. In this case, the third unrearranged variable region and the fourth unrearranged variable region may be lambda hetero-variable regions. In this case, the lambda hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the third human immunoglobulin lambda gene may be derived from a third human individual, and the fourth human immunoglobulin lambda gene may be derived from a fourth human individual. The third human individual and the fourth human individual may be different. Alternatively, the third human immunoglobulin lambda gene may be derived from an individual belonging to a third population(race), and the fourth human immunoglobulin lambda gene may be derived from an individual belonging to a fourth population(race). The third population(race) and the fourth population(race) may be different. In this case, the third population(race) may be the same as the first population(race). Alternatively, the first population(race), the second population(race), the third population(race), and the fourth population(race) may be different. In this case, each of the first population(race), the second population(race), the third population(race), and the fourth population(race) may be a population(race) selected from the group consisting of Caucasian (the Caucasoid race), Mongolian (the Mongoloid race), Negro (Negroid), the Malay race, Polynesian, and the Australoid race. Thus, the transgenic non-human animal may be a transgenic non-human animal having two alleles derived from different races for an immunoglobulin heavy chain gene and two alleles derived from different races for an immunoglobulin lambda gene. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In another embodiment, the transgenic animal may be a transgenic non-human animal having a genome including humanized immunoglobulin kappa gene loci (humanized IGK loci) and humanized immunoglobulin lambda gene loci (humanized IGL loci). The transgenic non-human animal may be a transgenic non-human animal having two alleles of each of a humanized immunoglobulin kappa gene (humanized IGK) and a humanized immunoglobulin lambda gene (humanized IGL). The humanized immunoglobulin kappa gene may include a variable region of a human immunoglobulin kappa gene and a constant region of a non-human animal immunoglobulin kappa gene. In this case, the variable region of the human immunoglobulin kappa gene may include unrearranged V and J segments. The two alleles of the humanized immunoglobulin kappa gene may be hetero-alleles. The hetero-alleles of the humanized immunoglobulin kappa gene may be a first humanized immunoglobulin kappa gene and a second humanized immunoglobulin kappa gene. In this case, the first humanized immunoglobulin kappa gene may include a first unrearranged variable region of a first human immunoglobulin kappa gene. The second humanized immunoglobulin kappa gene may include a second unrearranged variable region of a second human immunoglobulin kappa gene. In this case, the first unrearranged variable region and the second unrearranged variable region may be kappa hetero-variable regions. In this case, the kappa hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the first human immunoglobulin kappa gene may be derived from a first human individual, and the second human immunoglobulin kappa gene may be derived from a second human individual. The first human individual and the second human individual may be different. Alternatively, the first human immunoglobulin kappa gene may be derived from an individual belonging to a first population(race), and the second human immunoglobulin kappa gene may be derived from an individual belonging to a second population(race). The first population(race) and the second population(race) may be different. The humanized immunoglobulin lambda gene may include a variable region of a human immunoglobulin lambda gene and a constant region of a non-human animal immunoglobulin lambda gene. In this case, the variable region of the human immunoglobulin lambda gene may include unrearranged V and J segments. The two alleles of the humanized immunoglobulin lambda gene may be hetero-alleles. The hetero-alleles of the humanized immunoglobulin lambda gene may be a third humanized immunoglobulin lambda gene and a fourth humanized immunoglobulin lambda gene. In this case, the third humanized immunoglobulin lambda gene may include a third unrearranged variable region of a third human immunoglobulin lambda gene. The fourth humanized immunoglobulin lambda gene may include a fourth unrearranged variable region of a fourth human immunoglobulin lambda gene. In this case, the third unrearranged variable region and the fourth unrearranged variable region may be lambda hetero-variable regions. In this case, the lambda hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the third human immunoglobulin lambda gene may be derived from a third human individual, and the fourth human immunoglobulin lambda gene may be derived from a fourth human individual. The third human individual and the fourth human individual may be different. Alternatively, the third human immunoglobulin lambda gene may be derived from an individual belonging to a third population(race), and the fourth human immunoglobulin lambda gene may be derived from an individual belonging to a fourth population(race). The third population(race) and the fourth population(race) may be different. In this case, the third population(race) may be the same as the first population(race). Alternatively, the first population(race), the second population(race), the third population(race), and the fourth population(race) may be different. In this case, each of the first population(race), the second population(race), the third population(race) and the fourth population(race) may be a population(race) selected from the group consisting of Caucasian (the Caucasoid race), Mongolian (the Mongoloid race), Negro (Negroid), the Malay race, Polynesian, and the Australoid race. Thus, the transgenic non-human animal may be a transgenic non-human animal having two alleles derived from different races for an immunoglobulin kappa gene and two alleles derived from different races for an immunoglobulin lambda gene. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In one embodiment, the transgenic animal may be a transgenic non-human animal having a genome including humanized immunoglobulin heavy chain gene loci (humanized IGH loci), humanized immunoglobulin kappa gene loci (humanized IGK loci), and humanized immunoglobulin lambda loci (humanized IGL loci). The transgenic non-human animal may be a transgenic non-human animal having two alleles of each of a humanized immunoglobulin heavy chain gene (humanized IGH), a humanized immunoglobulin kappa gene (humanized IGK), and a humanized immunoglobulin lambda gene (humanized IGL). The humanized immunoglobulin heavy chain gene may include a variable region of a human immunoglobulin heavy chain gene and a constant region of a non-human animal immunoglobulin heavy chain gene. In this case, the variable region of the human immunoglobulin heavy chain gene may include unrearranged V, D, and J segments. The two alleles of the humanized immunoglobulin heavy chain gene may be hetero-alleles. The hetero-alleles of the humanized immunoglobulin heavy chain gene may be a first humanized immunoglobulin heavy chain gene and a second humanized immunoglobulin heavy chain gene. In this case, the first humanized immunoglobulin heavy chain gene may include a first unrearranged variable region of a first human immunoglobulin heavy chain gene. The second humanized immunoglobulin heavy chain gene may include a second unrearranged variable region of a second human immunoglobulin heavy chain gene. In this case, the first unrearranged variable region and the second unrearranged variable region may be heavy chain hetero-variable regions. In this case, the heavy chain hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the first human immunoglobulin heavy chain gene may be derived from a first human individual, and the second human immunoglobulin heavy chain gene may be derived from a second human individual. The first human individual and the second human individual may be different. Alternatively, the first human immunoglobulin heavy chain gene may be derived from an individual belonging to a first population(race), and the second human immunoglobulin heavy chain gene may be derived from an individual belonging to a second population(race). The first population(race) and the second population(race) may be different. The humanized immunoglobulin kappa gene may include a variable region of a human immunoglobulin kappa gene and a constant region of a non-human animal immunoglobulin kappa gene. In this case, the variable region of the human immunoglobulin kappa gene may include unrearranged V and J segments. The two alleles of the humanized immunoglobulin kappa gene may be hetero-alleles. The hetero-alleles of the humanized immunoglobulin kappa gene may be a third humanized immunoglobulin kappa gene and a fourth humanized immunoglobulin kappa gene. In this case, the third humanized immunoglobulin kappa gene may include a third unrearranged variable region of a third human immunoglobulin kappa gene. The fourth humanized immunoglobulin kappa gene may include a fourth unrearranged variable region of a fourth human immunoglobulin kappa gene. In this case, the third unrearranged variable region and the fourth unrearranged variable region may be kappa hetero-variable regions. In this case, the kappa hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the third human immunoglobulin kappa gene may be derived from a third human individual, and the fourth human immunoglobulin kappa gene may be derived from a fourth human individual. The third human individual and the fourth human individual may be different. Alternatively, the third human immunoglobulin kappa gene may be derived from an individual belonging to a third population(race), and the fourth human immunoglobulin kappa gene may be derived from an individual belonging to a fourth population(race). The third population(race) and the fourth population(race) may be different. The humanized immunoglobulin lambda gene may include a variable region of a human immunoglobulin lambda gene and a constant region of a non-human animal immunoglobulin lambda gene. In this case, the variable region of the human immunoglobulin lambda gene may include unrearranged V and J segments. The two alleles of the humanized immunoglobulin lambda gene may be hetero-alleles. The hetero-alleles of the humanized immunoglobulin lambda gene may be a fifth humanized immunoglobulin lambda gene and a sixth humanized immunoglobulin lambda gene. In this case, the fifth humanized immunoglobulin lambda gene may include a fifth unrearranged variable region of a fifth human immunoglobulin lambda gene. The sixth humanized immunoglobulin lambda gene may include a sixth unrearranged variable region of a sixth human immunoglobulin lambda gene. In this case, the fifth unrearranged variable region and the sixth unrearranged variable region may be lambda hetero-variable regions. In this case, the lambda hetero-variable regions may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. In this case, the fifth human immunoglobulin lambda gene may be derived from a fifth human individual, and the sixth human immunoglobulin lambda gene may be derived from a sixth human individual. The fifth human individual and the sixth human individual may be different. Alternatively, the fifth human immunoglobulin lambda gene may be derived from an individual belonging to a fifth population(race), and the sixth human immunoglobulin lambda gene may be derived from an individual belonging to a sixth population(race). The fifth population(race) and the sixth population(race) may be different. In this case, two or more of the first population(race), the second population(race), the third population(race), the fourth population(race), the fifth population(race), and the sixth population(race) may be the same population(race). Alternatively, the first population(race), the second population(race), the third population(race), the fourth population(race), the fifth population(race), and the sixth population(race) may be different. In this case, each of the first population(race), the second population(race), the third population(race), the fourth population(race), the fifth population(race), and the sixth population(race) may be a population(race) selected from the group consisting of Caucasian (the Caucasoid race), Mongolian (the Mongoloid race), Negro (Negroid), the Malay race, Polynesian, and the Australoid race. Thus, the transgenic non-human animal may be a transgenic non-human animal having: i) two alleles derived from different races for an immunoglobulin heavy chain gene; ii) two alleles derived from different races for an immunoglobulin kappa gene; and iii) two alleles derived from different races for an immunoglobulin lambda gene. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

The present invention provides a method of producing a transgenic animal having a genome including a humanized immunoglobulin locus. In this case, the transgenic animal may be a transgenic non-human animal having two alleles of a humanized immunoglobulin gene. In this case, the two alleles may be hetero-alleles. The transgenic animal may be an animal other than humans, such as a mouse, a rat, a rabbit, a goat, a monkey, a cow, a pig, a camel, and a chicken.

According to the method, a transgenic animal may be produced using two or more transgenic animal cells having hetero-alleles of a humanized immunoglobulin gene.

The transgenic animal cells may be mammalian cells other than humans, or avian cells. For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In this case, the two or more transgenic animal cells having hetero-alleles of a humanized immunoglobulin gene may be produced using known methods such as a cloning method using a vector and a method using chromosome exchange.

In one embodiment, in the method, a transgenic animal may be produced using two or more transgenic animal cells having hetero-alleles of a humanized immunoglobulin gene. In this case, the method may use a somatic cell nuclear transfer (SCNT) method.

In another embodiment, in the method, a transgenic animal may be produced using two or more transgenic animal embryos having hetero-alleles of a humanized immunoglobulin gene. In this case, the method may include implanting each of the two or more animal embryos into the uteri of surrogate mothers to produce transgenic animals, and crossing the produced transgenic animals.

In another embodiment, in the method, a transgenic animal may be produced using two or more transgenic animal embryonic stem cells having hetero-alleles of a humanized immunoglobulin gene. In this case, the method may include implanting the embryonic stem cells into blastulae to produce chimeric blastocysts, and implanting the chimeric blastocysts into the uteri of surrogate mothers to produce transgenic animals.

The present invention provides a method of producing an antibody using a transgenic animal having a genome including a humanized immunoglobulin locus. In this case, the method may include injecting an antigen into the transgenic animal. Through the injection of the antigen, the transgenic animal can produce an antigen-specific antibody. In this case, the antigen-specific antibody may be produced by recombination (rearrangement) of a humanized immunoglobulin gene at a humanized immunoglobulin locus. The transgenic animal may be a non-human animal. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In one embodiment, the method of producing an antibody may include injecting an antigen into a heterozygous transgenic animal. In this case, the heterozygous transgenic animal may be a heterozygous transgenic animal having hetero-alleles of an immunoglobulin gene. The immunoglobulin gene may be a humanized immunoglobulin gene, and the heterozygous transgenic animal may be a heterozygous transgenic animal having hetero-alleles of the humanized immunoglobulin gene. The heterozygous transgenic animal may have a genome including a humanized immunoglobulin locus having hetero-alleles of a humanized immunoglobulin gene. In this case, the humanized immunoglobulin gene may be a humanized immunoglobulin heavy chain gene, a humanized immunoglobulin kappa gene, and/or a humanized immunoglobulin lambda gene. Through the injection of the antigen, the heterozygous transgenic animal can produce an antigen-specific antibody. In this case, the antigen-specific antibody may be produced by recombination (rearrangement) of any one allele of the hetero-alleles.

In another embodiment, the method of producing an antibody may include injecting an antigen into a heterozygous transgenic animal. In this case, the heterozygous transgenic animal may be a heterozygous transgenic animal having hetero-alleles of an immunoglobulin gene. The immunoglobulin gene may be a humanized immunoglobulin gene, and the heterozygous transgenic animal may be a heterozygous transgenic animal having hetero-alleles of the humanized immunoglobulin gene. The heterozygous transgenic animal may have a genome including a humanized immunoglobulin locus having hetero-alleles of a humanized immunoglobulin gene. In this case, the humanized immunoglobulin gene may be a humanized immunoglobulin heavy chain gene, a humanized immunoglobulin kappa gene, and/or a humanized immunoglobulin lambda gene. In this case, the hetero-alleles may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb. Through the injection of the antigen, the heterozygous transgenic animal may produce an antigen-specific antibody. In this case, the antigen-specific antibody may be produced by recombination (rearrangement) of any one allele of the hetero-alleles.

### ADVANTAGEOUS EFFECTS OF INVENTION

The present invention relates to a heterozygous transgenic animal having hetero-alleles of an immunoglobulin gene. More specifically, the immunoglobulin gene is a humanized immunoglobulin gene, and the heterozygous transgenic animal is a heterozygous transgenic animal having hetero-alleles of a humanized immunoglobulin gene. The heterozygous transgenic animal can be used in the production of human antibodies. In particular, the heterozygous transgenic animal can be used to secure the diversity of an antibody repertoire.

### BRIEF DESCRIPTION OF DRAWINGS

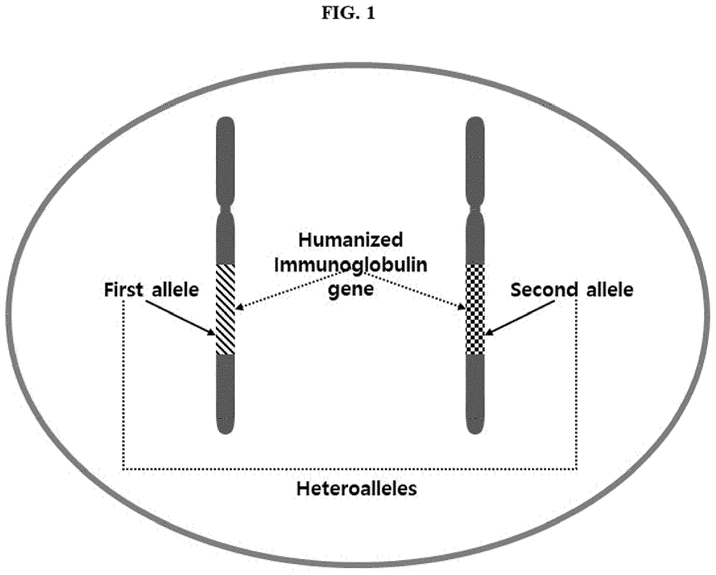

FIG. 1 is a view illustrating homologous chromosomes including a humanized immunoglobulin gene in the genome of a heterozygous transgenic animal having, as hetero-alleles, a humanized immunoglobulin gene derived from an individual belonging to a first race (a first allele) and a humanized immunoglobulin gene derived from an individual belonging to a second race (a second allele), wherein the first allele and the second allele are hetero-alleles, the two alleles are derived from different races, and the two alleles may have one or more of the following differences: i) synonymous mutation or non-synonymous mutation by SNP; ii) allelic variation of a V segment; iii) copy number variation (CNV) of a segment; iv) copy number variation (CNV) of an open reading frame (ORF); and v) deletion, insertion, or duplication of nucleic acids having a length of 8-75 kb.

### DETAILED DESCRIPTION OF INVENTION

### Definition of Terms

Definitions of terms used in the present specification are as follows.

### Homologous Chromosomes

"Homologous chromosomes" refer to a pair of chromosomes which are present in the nucleus of a cell and have the same shape and size. Genes for the same trait are present at the same position on two chromosomes, and are referred to as alleles.

### Alleles

"Alleles" are the smallest unit for expressing a specific trait in a genome and located at the same location on homologous chromosomes, i.e., at the same gene locus. Alleles are present in a pair for each gene in the autosome of a diploid organism with a 2n genome like humans, and the respective alleles are derived from a maternal line and a paternal line. The case where a pair of alleles are identical is referred to as homo-alleles, and the case where a pair of alleles are different is referred to as hetero-alleles.

In the present specification, the alleles of an immunoglobulin are alleles present in the germline, and include an unrearranged variable region.

The case where an allele includes a rearranged variable region, i.e., variable regions that V, D, and J segments or V and J segments are recombined is referred to as a recombinant allele.

### Gene Locus (Loci)

"Gene locus (loci)" means a fixed specific location on a chromosome where a specific gene is located. Haploid cells have one gene locus for a specific gene. Diploid cells have two gene loci for a specific gene. In this case, the two gene loci may include the same allele for a specific gene, i.e., homozygous alleles, respectively. Alternatively, the two gene loci may respectively include different alleles, i.e., heterozygous alleles, for a specific gene.

In addition, "gene locus" means a region where a specific gene is normally transcribed. That is, the gene locus means a region on a chromosome that includes all elements necessary for normal expression of a specific gene, such as exons, introns, promoters, enhancers, locus control regions (LCRs) and the like. For example, an immunoglobulin heavy locus (IGH) means a site where a gene of an immunoglobulin heavy chain is located on a chromosome. In addition, the immunoglobulin heavy locus (IGH) means a region including all elements necessary for normal expression of an immunoglobulin heavy-chain gene (promoters, enhancers, variable region gene, constant region gene, and the like).

### Immunoglobulin Heavy Locus (IGH Locus)

"Immunoglobulin heavy locus (IGH locus)" means a fixed location on a chromosome where an immunoglobulin heavy chain gene including variable (V), diversity (D), joining (J), and constant (C) segments is located. In addition, "immunoglobulin heavy locus (IGH locus)" means a region where normal transcription of an immunoglobulin heavy chain gene including variable (V), diversity (D), joining (J), and constant (C) segments occurs. The immunoglobulin heavy locus includes a recombined V-D-J region formed by recombination of the segments as B cells develop. As B cells develop, the segments included in an immunoglobulin heavy locus in the genome of the B cells during each development process may vary according to recombination. In contrast, a germline immunoglobulin heavy locus includes all of the V, D, J, and C segments in an unrecombined state, and the unrecombined V, D, and J segments are referred to as an "unrearranged variable region." The unrearranged variable region of the immunoglobulin heavy locus includes a plurality of V segments, a plurality of D segments, and a plurality of J segments. For example, in the case of humans, the unrearranged variable region of the immunoglobulin heavy locus includes 38 to 46 V segments, 23 D segments, and 6 J segments. In the case of mice, the unrearranged variable region of the immunoglobulin heavy locus includes 109 V segments, 19 D segments, and 4 J segments. In this case, each segment may have different alleles (segments) for each individual. Information on the alleles of some segments is summarized in the ImMunoGeneTics information system database (IMGT).

In the present specification, in the immunoglobulin heavy locus, the V segment is referred to as IGHV, the D segment is referred to as IGHD, and the J segment is referred to as IGHJ. In addition, the C segment of the constant region of the immunoglobulin heavy locus is referred to as IGHC.

### Immunoglobulin Lambda Locus (IGL locus)

"Immunoglobulin lambda locus (IGL locus)" means a fixed location on a chromosome where an immunoglobulin lambda gene including variable (V), joining (J), and constant (C) segments is located. In addition, "immunoglobulin lambda locus (IGL locus)" means a region where normal transcription of an immunoglobulin lambda gene including variable (V), joining (J), and constant (C) segments occurs. The immunoglobulin lambda locus includes a recombined V-J region formed by recombination of the segments as B cells develop. As B cells develop, the segments included in an immunoglobulin lambda locus in the genome of the B cells during each development process may vary according to recombination. In contrast, a germline immunoglobulin lambda locus includes all of the V, J, and C segments in an unrecombined state, and the unrecombined V and J segments are referred to as an "unrearranged variable region." The unrearranged variable region of the immunoglobulin lambda locus includes a plurality of V segments and a plurality of J segments. For example, in the case of humans, the unrearranged variable region of the immunoglobulin lambda locus includes 33 V segments and 5 J segments. In the case of mice, the unrearranged variable region of the immunoglobulin lambda locus includes 3 to 8 V segments and 4 J segments. In this case, each segment may have different alleles (segments) for each individual. Information on the alleles of some segments is summarized in the ImMunoGeneTics information system database (IMGT).

In the present specification, in the immunoglobulin lambda locus, the V segment is referred to as IGLV, and the J segment is referred to as IGLJ. In addition, the C segment of the constant region of the immunoglobulin lambda locus is referred to as IGLC.

### Immunoglobulin Kappa Locus (IGK locus)

"Immunoglobulin kappa locus (IGK locus)" means a fixed location on a chromosome where an immunoglobulin kappa gene including variable (V), joining (J), and constant (C) segments is located. In addition, "immunoglobulin kappa locus (IGK locus)" means a region where normal transcription of an immunoglobulin kappa gene including variable (V), joining (J), and constant (C) segments occurs. The immunoglobulin kappa locus includes a recombined V-J region formed by recombination of the segments as B cells develop. As B cells develop, the segments included in an immunoglobulin kappa locus in the genome of the B cells during each development process may vary according to recombination. In contrast, a germline immunoglobulin kappa locus includes all of the V, J, and C segments in an unrecombined state, and the unrecombined V and J segments are referred to as an "unrearranged variable region." The unrearranged variable region of the immunoglobulin kappa locus includes a plurality of V segments and a plurality of J segments. For example, in the case of humans, the unrearranged variable region of the immunoglobulin kappa locus includes 34 to 48 V segments and 5 J segments. In the case of mice, the unrearranged variable region of the immunoglobulin kappa locus includes 91 V segments and 4 J segments. In this case, each segment may have different alleles (segments) for each individual. Information on the alleles of some segments is summarized in the ImMunoGeneTics information system database (IMGT).

In this specification, in the immunoglobulin kappa locus, the V segment is referred to as IGKV and the J segment is referred to as IGKJ. In addition, the C segment of the constant region of the immunoglobulin kappa locus is referred to as IGKC.

### Humanized or Humanization

"Humanized or humanization" is a term meaning that the whole or part of a subject is that of a human or derived from a human, and when the term is used together with a gene, i.e., a humanized gene means that the whole or part of a nucleic acid sequence of a specific gene of a non-human animal is replaced with a nucleic acid sequence of a human gene or has the same nucleic acid sequence as that of a human gene. For example, the humanized immunoglobulin gene of a mouse may be a gene including a variable region of a human immunoglobulin gene instead of a variable region of a mouse immunoglobulin gene that is an endogenous immunoglobulin gene. Alternatively, the humanized immunoglobulin gene of a mouse may be a gene including a constant region of a human immunoglobulin gene instead of a constant region of a mouse immunoglobulin gene that is an endogenous immunoglobulin gene. Alternatively, the humanized immunoglobulin gene of a mouse may include a human immunoglobulin gene instead of a mouse immunoglobulin gene that is an endogenous immunoglobulin gene. Alternatively, the humanized immunoglobulin gene of a mouse may be a gene including a portion (a V segment) of the variable region of a human immunoglobulin gene instead of a portion (e.g., a V segment) of the variable region of a mouse immunoglobulin gene that is an endogenous immunoglobulin gene.

In addition, when the term is used together with a locus, i.e., a humanized locus refers to a state in which the whole or part of a nucleic acid sequence of a human gene is present in a specific locus of a non-human animal. For example, the humanized immunoglobulin locus of a rat may be a locus including a variable region of a human immunoglobulin gene in a rat immunoglobulin locus that is an endogenous immunoglobulin locus, instead of a variable region of an endogenous immunoglobulin gene. Alternatively, the humanized immunoglobulin locus of a rat may be a locus including a constant region of a human immunoglobulin gene in a rat immunoglobulin locus that is an endogenous immunoglobulin locus, instead of a constant region of an endogenous immunoglobulin gene. Alternatively, the humanized immunoglobulin locus of a rat may be a locus including a human immunoglobulin gene in a rat immunoglobulin locus that is an endogenous immunoglobulin locus, instead of an endogenous immunoglobulin gene. Alternatively, the humanized immunoglobulin locus of a rat may be a locus including a portion (J segment) of the variable region of a human immunoglobulin gene in a rat immunoglobulin locus that is an endogenous immunoglobulin locus, instead of a portion (e.g., a J segment) of the variable region of an endogenous immunoglobulin gene.

### Race

"Race (population)" is the concept of randomly dividing and classifying population groups that are perceived to have differences in terms of physical, social, and cultural characteristics among humans, and can be classified in various ways according to the classification method. In general, the method of classifying white, black, and yellow people based on morphological criteria such as skin color is most widely used. In the present specification, the race (population) is classified into, according to geographic and morphological criteria, Caucasian (the Caucasoid race), Mongolian (the Mongoloid race), Negro (Negroid), the Malay race, Polynesian, and the Australoid race.

Caucasians (or Caucasoid race) refer to white people distributed throughout Europe, North Africa, the Arabian Peninsula, Afghanistan, Northern India, South America, North America, and the like, who have features such as whilte skin, but some have brown skin, a wide forehead, a high nose, blue-brown or black eyes, and lots of body hair, but the morphological features are not limited thereto.

Mongolians (Mongoloid race) refer to yellow people distributed in Asia (East Asia, South Asia, West Asia, Asia Minor, Central Asia), Mongolia, East Siberia, Indochina, Hungary, Finland, and the like, who have features such as mainly yellow skin, but some have light brown skin, a wide forehead, a low nose, black straight hair, and little body hair, but the morphological features are not limited thereto.

Negros (Negroid) refer to black people distributed in Africa, North America, and the like, who have features such as coppery or dark brown skin, thick lips, a low nose, dark eyes, little body hair, and curly hair, but the morphological features are not limited thereto. Negros can be divided into the Congoid race (blacks in the South Pacific such as Australians) and the Capoid race (blacks native to southern Africa).

The Malay race is distributed in Indonesia, Philippines, New Guinea, Melanesia, and the like, which has brown skin and has features similar to those of Mongolians, but the morphological features are not limited thereto.

Polynesians (or the Australoid race) refer to a race distributed in the Pacific Islands such as Hawaii, Western Samoa, New Zealand, and Easter Island, which has features such as dark brown skin, a large body, and a large skull, but the morphological features are not limited thereto.

Unless otherwise defined, all technical and scientific terms used herein have the same meaning as those commonly understood by one of ordinary skill in the art to which the present invention pertains. Although methods and materials similar or identical to those described herein can be used in the practice or testing of the present invention, suitable methods and materials will be described below. All publications, patents, and other references mentioned herein are incorporated by reference in their entirety. Additionally, the materials, methods, and examples are illustrative only and are not intended to limit the present invention.

### An embodiment disclosed by the present specification relates to a heterozygous transgenic animal having hetero-alleles of an immunoglobulin gene.

More specifically, the immunoglobulin gene is a humanized immunoglobulin gene, and the heterozygous transgenic animal is a heterozygous transgenic animal having hetero-alleles of a humanized immunoglobulin gene. In this case, the heterozygous transgenic animal has a genome including a humanized immunoglobulin locus having hetero-alleles of a humanized immunoglobulin gene. The heterozygous transgenic animal may be used in the production of human antibodies. In particular, the heterozygous transgenic animal may be used to secure the diversity of an antibody repertoire.

### Antibody Repertoire

In one individual, there are approximately 10⁹ B cell libraries, and each clone can produce antibodies that specifically recognize different antigens. Among antibody diversity mechanisms, there are various recombinations (or rearrangements) of V, D, and J segments in an immunoglobulin heavy chain gene, and V and J segments in an immunoglobulin light chain gene. Pairing of various combinations of rearranged heavy and light chains are also included. Other mechanisms include somatic mutation, and removal or insertion of nucleotides at segment junctions when assembling segments. The antibody repertoire is the entire set of antibodies produced by these various mechanisms, and generally refers to the entire set of antibodies produced by an individual. Thus, securing the diversity of the antibody repertoire may increase the diversity of antibodies that specifically recognize an antigen. In addition, securing the diversity of the antibody repertoire may mean securing an antibody with increased specificity for an antigen. The diversity of the antibody repertoire may be secured by allelic exclusion and the diversification of immunoglobulin alleles (e.g., using differences in alleles between individuals and races). To this end, there is a need to develop a heterozygous transgenic animal for an immunoglobulin gene, not a homozygous transgenic animal.

### Homo-Allelic Immunoglobulin

Transgenic animals currently used to produce human antibodies are mostly homozygous transgenic animals having homo-alleles of an immunoglobulin gene. That is, transgenic animals having homo-alleles, as a pair of alleles, of an immunoglobulin gene are used in human antibody research and production. In this case, numerous patents such as US8,502,018, US9,371,553, US9,379,699, US9,447,177, US10,064,398, US9,504,236, US9,783,593, US9,445,581, US9,938,357, and US9,788,534 disclose the production of a homozygous transgenic mouse for an immunoglobulin gene and a method of producing a human antibody using the same. However, in the case of these homozygous transgenic mice, since an immunoglobulin gene is present as a homo-allele, germline gene diversity is relatively low compared to hetero-alleles, and thus B cell repertoire diversity, which can be produced by recombination (rearrangement) from alleles according to allelic exclusion, is limited.

### Allelic Exclusion of Immunoglobulin Gene

Allelic exclusion refers to a phenomenon in which only one of the two alleles of a diploid is expressed exclusively and functions normally and the other allele is not expressed. The allelic exclusion phenomenon can cause only one allele to work in two ways. The first is to regulate the transcription of only one of the two alleles by chromatin remodeling. The second is to produce a protein in which both alleles are transcribed, but only the mRNA derived from one allele functions normally. The most well-known allelic exclusion phenomenon is the process by which B cells (B lymphocytes) express only one type of B cell receptor (BCR).

In general, immunoglobulin genes are rearranged by randomly selecting one of the two alleles during the development of B cells. The substantially expressed genotype is one of the alleles, and when rearrangement of one allele proceeds normally, rearrangement of the other allele does not proceed. Alternatively, when rearrangement of one allele is not performed normally, rearrangement of the remaining allele proceeds. This allelic exclusion phenomenon of immunoglobulin genes ensures that one B cell has specificity for only one antigen.

This allelic exclusion of immunoglobulin genes is important in securing the diversity of an antibody repertoire. During the allele exclusion process, recombination of the V, D and J segments or V and J segments of an immunoglobulin gene occurs, and accordingly, through various recombinations, the diversity of the antibody repertoire is secured. In particular, according to germline gene diversity (depending on the presence or absence of homo-alleles or hetero-alleles), a difference in the diversity of a B cell repertoire formed by allelic exclusion may occur.

### B Cell Repertoire Diversity and Germline Gene Diversity

B cell repertoire diversity is attributed to the diversity of a plurality of V, D, and/or J segments included in an unrearranged variable region before the immunoglobulin gene is rearranged through the development of B cells. More specifically, B cell repertoire diversity is generally due to the diversity of alleles inherited from paternal and maternal lines. Germline gene diversity is due to the diversity of alleles inherited from paternal and maternal lines, i.e., diversity according to differences in some V, D, and/or J segments. This germline gene diversity plays an important role in B cell repertoire diversity. For example, germline gene diversity may increase the number of rearrangements of V, D, and/or J segments during the development of B cells. As described above, according to allelic exclusion of the immunoglobulin gene, one of the two alleles is randomly selected and rearranged during the development of B cells. The substantially expressed genotype is one of the alleles, and when rearrangement of one allele proceeds normally, rearrangement of the other allele does not proceed. Alternatively, when rearrangement of one allele is not performed normally, rearrangement of the remaining allele proceeds. Accordingly, a difference may occur in B cell repertoire diversity formed according to whether alleles are homo-alleles or hetero-alleles. In particular, when the alleles are hetero-alleles, B cell repertoire diversity may increase compared to the case of homo-alleles. This is because, in the case of hetero-alleles, the diversity of a plurality of V, D, and/or J segments included in an unrearranged variable region occur, thus increasing germline gene diversity. Therefore, there is a need to increase germline gene diversity in order to secure B cell repertoire diversity when human antibodies are produced using transgenic animals, and to this end, hetero-alleles may be effectively used.

### Hetero-Allelic Immunoglobulin

Existing transgenic animals for antibody production are homozygous transgenic animals having homo-alleles of an immunoglobulin gene, and the diversity of an antibody repertoire is limited. In order to secure the diversity of the antibody repertoire, there is a need to develop a heterozygous transgenic animal having hetero-alleles of an immunoglobulin gene. Transgenic animals having hetero-alleles of an immunoglobulin gene may have increased germline diversity. This is attributed to the difference in hetero-alleles. In addition, the difference in hetero-alleles may affect the diversity of the antibody repertoire.

### Difference between Hetero-alleles

In general, the alleles of immunoglobulin gene of mammals are hetero-alleles inherited from paternal and maternal lines, respectively. The difference in two alleles varies from a single-nucleotide polymorphism (SNP) to the deletion, insertion, or duplication of some regions of a gene (Chimge, N. O. et al. (2005) "Determination of gene organization in the human IGHV region on single chromosomes." Genes Immun. 6, 186-193; Li, H. et al. (2002) "Genetic diversity of the human immunoglobulin heavy chain VH region." Immunol. Rev. 190, 53-68; Kidd, M.J. et al. (2012) "The inference of phased haplotypes for the immunoglobulin H chain V region gene loci by analysis of VDJ gene rearrangements." J. Immunol. 188, 1333-1340; and Watson, C.T. et al. (2013) "Complete haplotype sequence of the human immunoglobulin heavy-chain variable, diversity, and joining genes and characterization of allelic and copy-number variation." Am. J.Hum.Genet. 92, 530-546).

Such a difference is seen not only in two alleles in an individual, but also between individuals, and the difference may vary more depending on the association or relationship between individuals (Watson, C.T. et al. (2014) "Sequencing of the human IG light chain loci from a hydatidiform mole BAC library reveals locus-specific signatures of genetic diversity." Genes Immun. 16, 24-23; Pallarès, N. et al. (1999) "The human immunoglobulin heavy variable genes." Exp. Clin. Immunogenet. 16, 36-60; Lefranc, M.-P. et al. (2014) "IMGT®, the international ImMunoGeneTics information system® 25 years on." Nucleic Acids Res. 43, D413-D422; Pallarés, N. et al. (1998) "The human immunoglobulin lambda variable (IGLV) genes and joining (IGLJ) segments." Exp. Clin. Immunogenet. 15, 8-18; and Barbi6, V and Lefranc, M.P. (1998) "The human immunoglobulin kappa variable (IGKV) genes and joining (IGKJ) segments." Exp. Clin. Immunogenet. 15, 171-183).

For example, two different individuals show a significant difference in the diversity of the antibody repertoire, which is a result of differences in allelic variation of a V segment and polymorphism within the V segment (Boyd, S.D. et al. (2010) "Individual variation in the germline Ig gene repertoire inferred from variable region gene rearrangements." J. Immunol. 184, 6986-6992).

As another example, it was confirmed that the alleles of an immunoglobulin gene of an individual who is Caucasian (or the Caucasoid race) show a significant difference from the alleles of an immunoglobulin gene of an individual who is Negro (Negroid) (Scheepers, C. et al. (2015) "Ability to develop broadly neutralizing HIV-1 antibodies is not restricted by the germline IG gene repertoire." J. Immunol. 194, 4371-4378; and Wang, Y. et al. (2011) "Genomic screening by 454 pyrosequencing identifies a new human IGHV gene and sixteen other new IGHV allelic variants." Immunogenetics 63, 259-265). In particular, it has been reported in numerous papers that genetic differences between races are diverse (Mallick, Swapan, et al. (2016) "The Simons genome diversity project: 300 genomes from 142 diverse populations." Nature 538, 201-206). Thus, when alleles derived from different races are used, B cell repertoire diversity may increase due to the diversity of alleles, and various novel antibodies may be derived.

### Hetero-alleles derived from Different Individuals or Different Races

The use of hetero-alleles makes it possible to secure the diversity of an antibody repertoire. In particular, depending on the difference in hetero-alleles, the diversity of the antibody repertoire may vary. The greater the difference between hetero-alleles, the greater the diversity of the antibody repertoire produced. To this end, two alleles derived from different individuals (human individuals) may be used in a transgenic animal for antibody production. Specifically, human antibodies may be produced using a transgenic animal having, as hetero-alleles, a humanized immunoglobulin gene (first allele) derived from a first human individual and a humanized immunoglobulin gene (second allele) derived from a second human individual. In this case, the first human individual and the second human individual may be different. In this case, the first allele and the second allele show a difference in an unrearranged variable region. In this case, the unrearranged variable region between the hetero-alleles may have the following differences (Watson CT et al. (2017) "The individual and population genetics of antibody immunity." Trends Immunol. 38(7), 459-470):
i) synonymous mutation or non-synonymous mutation by a single nucleotide polymorphism (SNP);
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

Alternatively, two alleles from different races may be used in a transgenic animal for antibody production. Specifically, human antibodies may be produced using a transgenic animal having, as hetero-alleles, a humanized immunoglobulin gene (first allele) derived from an individual belonging to a first race and a humanized immunoglobulin gene (second allele) derived from an individual belonging to a second race. In this case, the first allele and the second allele show a difference in an unrearranged variable region. In this case, the unrearranged variable region between the hetero-alleles may have the following differences (Watson CT et al. (2017) "The individual and population genetics of antibody immunity." Trends Immunol. 38(7), 459-470):
i) synonymous mutation or non-synonymous mutation by a single nucleotide polymorphism (SNP);
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

The difference between hetero-alleles as described above may increase antibody repertoire diversity by securing germline gene diversity.

### i) Synonymous Mutation or Non-synonymous Mutation by Single Nucleotide Polymorphism (SNP)

A single nucleotide polymorphism (SNP) is a genetic change or mutation that shows a difference in one nucleotide sequence in a DNA sequence, and usually occurs once every 1,000 nucleotides in the genome, resulting in genetic diversity. The synonymous mutation by SNP refers to a mutation in which SNPs are present in the genome, but there is no change in amino acids expressed thereby. In the case of synonymous mutations, when a point mutation occurs in the genome, that is, in DNA, there is also a mutation in the transcribed RNA sequence accordingly, but there is no mutation in the translated amino acid. In contrast, the non-synonymous mutation by SNP refers to a mutation in which a SNP is present by substitution, insertion, or deletion of one nucleotide in the genome, and by this SNP, the codon sequence is changed or a frame-shift mutation occurs, and thus the expressed amino acid is modified. In the case of non-synonymous mutation, when a mutation occurs by substitution, insertion, or deletion of one nucleotide in the genome, i.e., in a DNA sequence, the reading frame of a codon sequence is changed, and thus the expressed protein or amino acid is modified.

### ii) Allelic Variation of V segment

Allelic variation of a V segment refers to including one or more polymorphism mutations in some of the V segments present in the variable region of an immunoglobulin. For example, V1-69 segments among the V segments in the variable region of an immunoglobulin heavy chain gene have alleles such as IGHV1-69*01, IGHV1-69*02, IGHV1-69*03, IGHV1-69*04, IGHV1-69^{∗}05, IGHV1-69*06, IGHV1-69*07, IGHV1-69*08, IGHV1-69*09, IGHV1-69*10, IGHV1-69*11, IGHV1-69*12, IGHV1-69*13 and the like.

### iii) Copy Number Variation (CNV) of Segment

The copy number variation of a segment refers to deletion or repetition (duplication) of some of the V, J and/or D segments constituting the variable region of an immunoglobulin. For example, D1-14 segments among the D segments in the variable region of an immunoglobulin heavy chain gene may be duplicated, and in this case, the immunoglobulin heavy chain gene may be said to have a copy number variation of the segment. As another example, V2-28 segments among the V segments in the variable region of an immunoglobulin kappa gene may be deleted, and in this case, the immunoglobulin kappa gene may be said to have a copy number variation of the segment.

### iv) Copy Number Variation (CNV) of Open Reading Frame (ORF)

The open reading frame (ORF) refers to a nucleotide sequence that has the potential to be transcribed into mRNA and translated into a protein. ORF copy number variation refers to the deletion or repetition (duplication) of some ORFs in the immunoglobulin gene.

### v) Deletion, Insertion or Duplication of Nucleic Acids having Length of 8-75 kb

Immunoglobulin genes can be modified by deletion, insertion, or duplication of large nucleic acid segments. For example, a region of the immunoglobulin gene may be deleted or duplicated. Alternatively, one region may be inserted into the immunoglobulin gene. In this case, the one region may be a large nucleic acid sequence of at least 8-75 kb in length. Variations generated by deletion, insertion, or duplication of such large nucleic acid fragements may cause a copy number variation of a specific segment.

### Hetero-allelic Immunoglobulin Heavy (IGH)

In one embodiment, the heterozygous transgenic animal disclosed in the present specification may have a genome including hetero-alleles of a humanized immunoglobulin heavy chain gene in order to secure the diversity of an antibody repertoire. The hetero-alleles may be two alleles derived from different individuals. In this case, the different individuals may be individuals belonging to different races. For example, the hetero-alleles may be a humanized immunoglobulin heavy chain gene (first allele) derived from an individual belonging to a first race and a humanized immunoglobulin heavy chain gene (second allele) derived from an individual belonging to a second race. The first race and the second race may be different, and each of the first race and the second race may be a race selected from the group consisting of Caucasian (the Caucasoid race), Mongolian (the Mongoloid race), Negro (Negroid), the Malay race, Polynesian, and the Australoid race. The first allele and the second allele may have one or more of the following differences:
i) synonymous mutation or non-synonymous mutation by SNP;
ii) allelic variation of an IGHV segment;
iii) copy number variation (CNV) of a segment;
iv) copy number variation (CNV) of an open reading frame (ORF); and
v) deletion, insertion, or duplication of nucleic acids having a length of 8-75 kb.

### Hetero-allelic Immunoglobulin Kappa (IGK)

In one embodiment, the heterozygous transgenic animal disclosed in the present specification may have a genome including hetero-alleles of a humanized immunoglobulin kappa gene in order to secure the diversity of an antibody repertoire. The hetero-alleles may be two alleles derived from different individuals. In this case, the different individuals may be different human individuals. In this case, the different individuals may be individuals belonging to different races. For example, the hetero-alleles may be a humanized immunoglobulin kappa gene (first allele) derived from an individual belonging to a first race and a humanized immunoglobulin kappa gene (second allele) derived from an individual belonging to a second race. The first race and the second race may be different, and each of the first race and the second race may be a race selected from the group consisting of Caucasian (the Caucasoid race), Mongolian (the Mongoloid race), Negro (Negroid), the Malay race, Polynesian, and the Australoid race. The first allele and the second allele may have one or more of the following differences:
i) synonymous mutation or non-synonymous mutation by SNP;
ii) allelic variation of an IGKV segment;
iii) copy number variation (CNV) of a segment;
iv) copy number variation (CNV) of an open reading frame (ORF); and
v) deletion, insertion, or duplication of nucleic acids having a length of 8-75 kb.

### Hetero-allelic Immunoglobulin Lambda (IGL)

In one embodiment, the heterozygous transgenic animal disclosed in the present specification may have a genome including hetero-alleles of a humanized immunoglobulin lambda gene in order to secure the diversity of an antibody repertoire. The hetero-alleles may be two alleles derived from different individuals. In this case, the different individuals may be different human individuals. In this case, the different individuals may be individuals belonging to different races. For example, the hetero-alleles may be a humanized immunoglobulin lambda gene (first allele) derived from an individual belonging to a first race and a humanized immunoglobulin lambda gene (second allele) derived from an individual belonging to a second race. The first race and the second race may be different, and each of the first race and the second race may be a race selected from the group consisting of Caucasian (the Caucasoid race), Mongolian (the Mongoloid race), Negro (Negroid), the Malay race, Polynesian, and the Australoid race. The first allele and the second allele may have one or more of the following differences:
i) synonymous mutation or non-synonymous mutation by SNP;
ii) allelic variation of an IGLV segment;
iii) copy number variation (CNV) of a segment;
iv) copy number variation (CNV) of an open reading frame (ORF); and
v) deletion, insertion, or duplication of nucleic acids having a length of 8-75 kb.

### Hetero-allelic Immunoglobulin heavy (IGH), Hetero-allelic Immunoglobulin Kappa (IGK), and/or Hetero-allelic Immunoglobulin Lambda (IGL)

In one embodiment, the heterozygous transgenic animal disclosed herein may have a genome including hetero-alleles of each of a humanized immunoglobulin heavy chain gene and a humanized immunoglobulin kappa gene in order to secure the diversity of an antibody repertoire. The hetero-alleles may be two alleles derived from different individuals. In this case, the different individuals may be different human individuals. In this case, the different individuals may be individuals belonging to different races. For example, the hetero-alleles of the humanized immunoglobulin heavy chain gene may be a humanized immunoglobulin heavy chain gene (first heavy chain allele) derived from an individual belonging to a first race and a humanized immunoglobulin heavy chain gene (second heavy chain allele) derived from an individual belonging to a second race. The first race and the second race may be different. The hetero-alleles of the humanized immunoglobulin kappa gene may be a humanized immunoglobulin kappa gene (first kappa allele) derived from an individual belonging to a third race and a humanized immunoglobulin kappa gene (second kappa allele) derived from an individual belonging to a fourth race. The third race and the fourth race may be different. In this case, the first race may be the same as the third race or the fourth race. Each of the first race, the second race, the third race, and the fourth race may be a race selected from the group consisting of Caucasian (the Caucasoid race), Mongolian (the Mongoloid race), Negro (Negroid), the Malay race, Polynesian, and the Australoid race. Each of the first heavy chain allele and the second heavy chain allele, and each of the first kappa allele and the second kappa allele may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

In another embodiment, the heterozygous transgenic animal disclosed herein may have a genome including hetero-alleles of each of a humanized immunoglobulin heavy chain gene and a humanized immunoglobulin lambda gene in order to secure the diversity of an antibody repertoire. The hetero-alleles may be two alleles derived from different individuals. In this case, the different individuals may be different human individuals. In this case, the different individuals may be individuals belonging to different races. For example, the hetero-alleles of the humanized immunoglobulin heavy chain gene may be a humanized immunoglobulin heavy chain gene (first heavy chain allele) derived from an individual belonging to a first race and a humanized immunoglobulin heavy chain gene (second heavy chain allele) derived from an individual belonging to a second race. The first race and the second race are different. The hetero-alleles of the humanized immunoglobulin lambda gene may be a humanized immunoglobulin lambda gene (first lambda allele) derived from an individual belonging to a third race and a humanized immunoglobulin lambda gene (second lambda allele) derived from an individual belonging to a fourth race. The third race and the fourth race are different. In this case, the first race may be the same as the third race or the fourth race. Each of the first race, the second race, the third race, and the fourth race may be a race selected from the group consisting of Caucasian (the Caucasoid race), Mongolian (the Mongoloid race), Negro (Negroid), the Malay race, Polynesian, and the Australoid race. Each of the first heavy chain allele and the second heavy chain allele, and each of the first lambda allele and the second lambda allele may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

In another embodiment, the heterozygous transgenic animal disclosed herein may have a genome including hetero-alleles of each of a humanized immunoglobulin kappa gene and a humanized immunoglobulin lambda gene in order to secure the diversity of an antibody repertoire. The hetero-alleles may be two alleles derived from different individuals. In this case, the different individuals may be different human individuals. In this case, the different individuals may be individuals belonging to different races. For example, the hetero-alleles of the humanized immunoglobulin kappa gene may be a humanized immunoglobulin kappa gene (first kappa allele) derived from an individual belonging to a first race and a humanized immunoglobulin kappa gene (second kappa allele) derived from an individual belonging to a second race. The first race and the second race are different. The hetero-alleles of the humanized immunoglobulin lambda gene may be a humanized immunoglobulin lambda gene (first lambda allele) derived from an individual belonging to a third race and a humanized immunoglobulin lambda gene (second lambda allele) derived from an individual belonging to a fourth race. The third race and the fourth race are different. In this case, the first race may be the same as the third race or the fourth race. Each of the first race, the second race, the third race, and the fourth race may be a race selected from the group consisting of Caucasian (the Caucasoid race), Mongolian (the Mongoloid race), Negro (Negroid), the Malay race, Polynesian, and the Australoid race. Each of the first kappa allele and the second kappa allele, and each of the first lambda allele and the second lambda allele may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

In another embodiment, the heterozygous transgenic animal disclosed herein may have a genome including hetero-alleles of each of a humanized immunoglobulin heavy chain gene, a humanized immunoglobulin kappa gene, and a humanized immunoglobulin lambda gene in order to secure the diversity of an antibody repertoire. The hetero-alleles may be two alleles derived from different individuals. In this case, the different individuals may be different human individuals. In this case, the different individuals may be individuals belonging to different races. For example, the hetero-alleles of the humanized immunoglobulin heavy chain gene may be a humanized immunoglobulin heavy chain gene (first heavy chain allele) derived from an individual belonging to a first race and a humanized immunoglobulin heavy chain gene (second heavy chain allele) derived from an individual belonging to a second race. The first race and the second race are different. The hetero-alleles of the humanized immunoglobulin kappa gene may be a humanized immunoglobulin kappa gene (first kappa allele) derived from an individual belonging to a third race and a humanized immunoglobulin kappa gene (second kappa allele) derived from an individual belonging to a fourth race. The third race and the fourth race are different. The hetero-alleles of the humanized immunoglobulin lambda gene may be a humanized immunoglobulin lambda gene (first lambda allele) derived from an individual belonging to a fifth race and a humanized immunoglobulin lambda gene (second lambda allele) derived from an individual belonging to a sixth race. The fifth race and the sixth race are different. In this case, two or more races selected from the first race, the second race, the third race, the fourth race, the fifth race, and the sixth race may be the same. Each of the first race, the second race, the third race, the fourth race, the fifth race, and the sixth race may be a race selected from the group consisting of Caucasian (the Caucasoid race), Mongolian (the Mongoloid race), Negro (Negroid), the Malay race, Polynesian, and the Australoid race. Each of the first heavy chain allele and the second heavy chain allele, each of the first kappa allele and the second kappa allele, and each of the first lambda allele and the second lambda allele may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

In this case, the transgenic animal is a non-human animal, and the non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

### Increase in Antibody Repertoire Diversity by Hetero-alleles derived from Different Individuals (or Races)

Germline gene diversity according to hetero-alleles increases B cell repertoire diversity. Generally, one of the two alleles of an immunoglobulin gene is randomly selected and expressed (Judith A. Owen, Jenni Punt, Sharon A. Stranford, (2013) Kuby Immunology (7th edition), Chapter 7, W. H. Freeman & Company). In other words, when a VDJ combination or a VJ combination is normally formed by recombination (or rearrangement) of V, D, and J segments, or V and J segments occurring in one allele (e.g., a first allele) selected from the two alleles (first allele and second allele) of an immunoglobulin gene, the second allele is not expressed and only the recombined first allele is expressed. However, when a normal VDJ combination or VJ combination is not formed in the first allele, the recombination (or rearrangement) of V, D, and J segements or V and J segments occurs in the second allele. When a normal VDJ combination or VJ combination is formed in the second allele, the first allele is not expressed and only the normally recombined second allele is expressed. These traits are commonly shown in immunoglobulin heavy chain, kappa, and lambda genes. In general, it is known that the recombination (or rearrangement) of V, D, and J segements or V and J segments occurs in the order of immunoglobulin heavy chain, kappa, and lambda genes.

When hetero-alleles are used, the expression characteristics of a single allele according to the recombination (rearrangement) of an immunoglobulin gene may increase the diversity of recombination compared to the case where homo-alleles are used. In other words, since the number of recombinations that occurs by two hetero-alleles is much greater than the number of recombinations that may occur by two homo-alleles, antibody repertoire diversity may be increased by hetero-alleles.

In addition, some nucleotides are deleted or inserted at a segment junction upon recombination (or rearrangement) of V, D, and J segments or V and J segments. This deletion or insertion of nucleotides leads to junctional diversity (Judith A. Owen, Jenni Punt, Sharon A. Stranford, (2013) Kuby Immunology (7th edition), Chapter 7, W. H. Freeman & Company). Such junctional diversity may increase antibody repertoire diversity along with the recombination (rearrangement) of an immunoglobulin gene.

In addition, the diversity of the antibody repertoire may be increased by somatic hypermutation and class switching (Judith A. Owen, Jenni Punt, Sharon A. Stranford, (2013) Kuby Immunology (7th edition), Chapter 12, W. H. Freeman & Company).

Thus, the antibody repertoire diversity may be increased using hetero-alleles compared to homo-alleles. This is due to, by hetero-alleles, an increase in the diversity of a V, D, or J segment, an increase in the number of recombinations (or rearrangements) of V, D, and J segments or V and J segments, increases in segment diversity and junctional diversity according to increased recombination, and an increase in diversity by somatic hypermutation and/or class switching in the increased VDJ combination or VJ combination.

### Another embodiment disclosed in the present specification relates to a method of producing a heterozygous transgenic animal having hetero-alleles of an immunoglobulin gene.

More specifically, the immunoglobulin gene is a humanized immunoglobulin gene, and the heterozygous transgenic animal is a heterozygous transgenic animal having hetero-alleles of a humanized immunoglobulin gene. In this case, the heterozygous transgenic animal has a genome including a humanized immunoglobulin locus having hetero-alleles of a humanized immunoglobulin gene. The heterozygous transgenic animal may be used in the production of human antibodies. In particular, the heterozygous transgenic animal may be used to secure the diversity of an antibody repertoire.

The heterozygous transgenic animal may be produced using two or more transgenic animal cells having hetero-alleles of a humanized immunoglobulin gene.

In this case, the two or more transgenic animal cells having hetero-alleles of a humanized immunoglobulin gene may be produced using known methods such as a cloning method using a vector and a method using chromosome exchange. For example, for the cloning method using a vector, US 6,586,251, US 6,596,541, US 7,105,348, and US 2004-0018626 A1 can be referenced, but the present invention is not limited thereto. For example, for the method using chromosome exchange, PCT/KR2019/015351 and KR 10-2019-0042840 can be referenced, but the present invention is not limited thereto.

The heterozygous transgenic animal may be a non-human animal other than humans. The non-human animal may be a mammal other than humans, or a bird (aves). For example, the mammal may be a rodent, an ungulate, or a non-human primate. In this case, the rodent may be a mouse, a rat, or the like, the ungulate may be a rabbit, a goat, a cow, a pig, a camel, or the like, the non-human primate may be a chimpanzee, a monkey, or the like, and the aves may be a chicken, a quail, or the like, but the present invention is not limited thereto.

In one embodiment, the two or more transgenic animal cells having hetero-alleles of a humanized immunoglobulin gene may be produced using a chromosome exchange (or replacement, substitution) technique. In this case, the two or more transgenic animal cells may be a first transgenic animal cell and a second transgenic animal cell.

The first transgenic animal cell may be produced using the following method:
a) preparing a human cell derived from an individual belonging to a first race and a non-human animal cell;
b) producing a microcell using the human cell derived from an individual belonging to a first race;
c) producing a fusion non-human animal cell using the microcell and the non-human animal cell; and
d) producing a first transgenic animal cell having a humanized immunoglobulin gene through recombinase treatment.

### In process a),

The human cell derived from an individual belonging to a first race is a cell manipulated such that recombination elements (e.g., loxp, FRT, attP, attB, and ITR) are included at both ends of the variable region of a human immunoglobulin locus, and the non-human animal cell may be a cell manipulated such that recombination elements (e.g., loxp, FRT, attP, attB, and ITR) are included at both ends of the variable region of a non-human animal immunoglobulin locus.

In this case, the recombinant elements at both ends of the variable region of the human immunoglobulin locus and the recombinant elements at both ends of the variable region of the non-human animal immunoglobulin locus may be paired with each other.

For example, the both ends of the variable region of the human immunoglobulin locus may include Lox66 and Loxm2/71 as recombinant elements, respectively, and the both ends of the variable region of the non-human animal immunoglobulin locus may include Loxm2/66 and Lox71 as recombinant elements, respectively. In this case, the Lox66 may be paired with the Lox71, and the Loxm2/71 may be paired with the Loxm2/66.

### In process b),

The microcell may be produced using the human cell derived from an individual belonging to a first race through a known method. This is described in the literature "Thorfinn Ege et al 1974" and "Thorfinn Ege et al 1977," which can be referred to.

### In process c),

The fusion non-human animal cell may be produced by bringing the microcell produced by process b) into contact with the non-human animal cell and fusing the same. This is described in the literature "Fournier RE et al 1977," "McNeill CA et al 1980," and "Tomizuka et al., Nature Genetics, 16: 133 (1997)," which can be referred to.

### In process d),

The fusion non-human animal cell produced by process c) may be treated with a recombinase to produce a first transgenic animal cell having a humanized immunoglobulin locus. For example, the recombinase may be Cre recombinase. In this case, the Cre recombinase may recognize recombination elements (e.g., Lox66 and Loxm2/71) at both ends of the variable region of the human immunoglobulin locus and recombination elements (e.g., Loxm2/66 and Lox71) at both ends of the variable region of the non-human animal immunoglobulin locus. In particular, Cre recombinase may induce recombination by recognizing the pairing of the recombination elements (e.g., pairing between Lox66 and Lox71, pairing between Loxm2/71 and Loxm2/66). Through such recombination, a humanized immunoglobulin locus may be produced, and the humanized immunoglobulin locus may include the variable region of a human immunoglobulin gene derived from an individual belonging to a first race and the constant region of a non-human animal immunoglobulin gene.

In addition, the second transgenic animal cell may be produced using the same method as the method described above used to produce the first transgenic animal cell. Here, instead of the human cell derived from an individual belonging to a first race, a human cell derived from an individual belonging to a second race may be used. In this case, the first race and the second race may be different. Each of the first race and the second race may be a race selected from the group consisting of Caucasian (the Caucasoid race), Mongolian (the Mongoloid race), Negro (Negroid), the Malay race, Polynesian, and the Australoid race.

For example, the first race may be Caucasian, and the second race may be Negro. In this case, in process a), the human cell derived from an individual belonging to a first race may be a BJ cell (ATCC^{®} CRL-2522^{™}, ethnicity is white), which is a human fibroblast derived from an individual who is Caucasian, and the human cell derived from an individual belonging to a second race may be a WS1 cell (ATCC^{®} CRL-1502^{™}, ethnicity is black), which is a human fibroblast derived from an individual who is Negro.

### Somatic Cell Nuclear Transfer (SCNT)

In one embodiment, the heterozygous transgenic animal may be produced through somatic cell nuclear transfer (SCNT) using two or more transgenic animal cells having hetero-alleles of a humanized immunoglobulin gene. In this case, the transgenic animal cells are two or more transgenic animal cells having humanized immunoglobulin genes derived from different individuals, and may include a first transgenic animal cell having a humanized immunoglobulin gene derived from a first individual and a second transgenic animal cell having a humanized immunoglobulin gene derived from a second individual. In this case, the humanized immunoglobulin gene of the first transgenic animal cell and the humanized immunoglobulin gene of the second transgenic animal cell may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

The somatic cell nuclear transfer involves obtaining a donor nucleus from each of the first transgenic animal cell and the second transgenic animal cell, implanting each donor nucleus into an enucleated egg to produce each cloned egg, and transplanting each cloned egg into the uterus of a surrogate mother to generate each living offspring, and the SCNT may use a known method. The produced living offsprings may be crossed to obtain a heterozygous transgenic animal.

### Generation from Embryo

In one embodiment, the heterozygous transgenic animal may be produced using two or more transgenic animal cells having hetero-alleles of a humanized immunoglobulin gene. In this case, the transgenic animal cells may be transgenic embryos. In this case, the transgenic embryos are two or more transgenic embryos having humanized immunoglobulin genes derived from different individuals, and may include a first transgenic embryo having a humanized immunoglobulin gene derived from a first individual and a second transgenic embryo having a humanized immunoglobulin gene derived from a second individual. In this case, the humanized immunoglobulin gene of the first transgenic embryo and the humanized immunoglobulin gene of the second transgenic embryo may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

The heterozygous transgenic animal may be obtained by implanting each of the first transgenic embryo and the second transgenic embryo into the uterus of a surrogate mother to give birth to a living offspring from each embryo, and crossing the obtained living offsprings, and a known method may be used.

### Blastocyst Injection

In one embodiment, the heterozygous transgenic animal may be produced through blastocyst injection using two or more transgenic animal cells having hetero-alleles of a humanized immunoglobulin gene. In this case, the transgenic animal cells may be transgenic embryonic stem cells (ES cells). In this case, the transgenic embryonic stem cells are two or more transgenic embryonic stem cells having humanized immunoglobulin genes derived from different individuals, and may include a first transgenic embryonic stem cell having a humanized immunoglobulin gene derived from a first individual and a second transgenic embryonic stem cell having a humanized immunoglobulin gene derived from a second individual. In this case, the humanized immunoglobulin gene of the first transgenic embryonic stem cell and the humanized immunoglobulin gene of the second transgenic embryonic stem cell may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

The heterozygous transgenic animal may be obtained by implanting each of the first transgenic embryonic stem cell and the second transgenic embryonic stem cell into a blastula to produce a chimeric blastocyst, implanting the chimeric blastocyst into the uterus of a surrogate mother to give birth to each living offspring, and crossing the obtained living offsprings, and a known method may be used.

### Another embodiment disclosed by the present specification relates to a method of producing an antibody using a heterozygous transgenic animal having hetero-alleles of an immunoglobulin gene. In this case, the method may include injecting an antigen into the heterozygous transgenic animal.

The immunoglobulin gene is a humanized immunoglobulin gene, and the heterozygous transgenic animal may be a heterozygous transgenic animal having hetero-alleles of a humanized immunoglobulin gene. In this case, the heterozygous transgenic animal may have a genome including a humanized immunoglobulin locus having hetero-alleles of a humanized immunoglobulin gene.

The humanized immunoglobulin gene may be a humanized immunoglobulin heavy chain gene, a humanized immunoglobulin kappa gene, and/or a humanized immunoglobulin lambda gene.

The hetero-alleles may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

Through the injection of the antigen, the heterozygous transgenic animal may produce an antigen-specific antibody. In this case, the antigen-specific antibody may be produced by recombination (rearrangement) of any one allele of the hetero-alleles.

### Another embodiment disclosed by the present specification relates to a method of increasing antibody diversity using a heterozygous transgenic animal having hetero-alleles of an immunoglobulin gene. In this case, the method may include injecting an antigen into the heterozygous transgenic animal.

The immunoglobulin gene is a humanized immunoglobulin gene, and the heterozygous transgenic animal may be a heterozygous transgenic animal having hetero-alleles of a humanized immunoglobulin gene. In this case, the heterozygous transgenic animal may have a genome including a humanized immunoglobulin locus having hetero-alleles of a humanized immunoglobulin gene.

The humanized immunoglobulin gene may be a humanized immunoglobulin heavy chain gene, a humanized immunoglobulin kappa gene, and/or a humanized immunoglobulin lambda gene.

The hetero-alleles may have one or more of the following differences:
i) synonymous mutation or non-synonymous mutation by SNP;
ii) allelic variation of a V segment;
iii) copy number variation (CNV) of a segment;
iv) copy number variation (CNV) of an open reading frame (ORF); and
v) deletion, insertion, or duplication of nucleic acids having a length of 8-75 kb.

Through the method, an antigen-specific antibody population or B cell population may be obtained from the heterozygous transgenic animal through the injection of the antigen. In this case, the antigen-specific antibody population or B cell population may be an antibody population or B cell population produced by recombination (rearrangement) of any one allele of the hetero-alleles.

Hereinafter, the present invention will be described in detail with reference to the following examples.

It will be obvious to those of ordinary skill in the art to which the present invention pertains that these examples are provided merely to more specifically explain the present invention, and the scope of the present invention is not limited by these examples.

### Example 1. Method of Producing Heterozygous Transgenic Mice using Chromosome Exchange

The present example relates to a method of producing a heterozygous transgenic mouse having a genome including a humanized immunoglobulin locus, and relates to a transgenic mouse having a humanized immunoglobulin gene, and having hetero-alleles of the humanized immunoglobulin gene. The following description is an overall example regarding the production of a humanized heterozygous transgenic mouse by substitution (or replacement) of the variable regions of immunoglobulin heavy loci of mice with the variable regions of human immunoglobulin genes derived individuals belonging to different races, and the production of a heterozygous transgenic mouse is merely an example, and the present invention is not limited thereto. A desired heterozygous transgenic mouse may be produced by making various changes in the examples described below, and may be produced using various methods other than the examples described below.

### Example 1-1. Method of Producing Homozygous Transgenic Mouse having Humanized Immunoglobulin Gene derived from Negro

The present example relates to the production of a humanized homozygous transgenic mouse by substitution (or replacement) of the variable region of an immunoglobulin heavy locus of a mouse with the variable region of a human immunoglobulin gene derived from an individual who is Negro. In the corresponding example, a homozygous transgenic mouse having a Negro-derived humanized immunoglobulin gene is produced using a chromosome exchange technique (e.g., AiCE technique (see PCT/KR2019/015351)).

### 1. Vector Construction

In order to substitute (or replace) the variable region of a mouse immunoglobulin gene with the variable region of a human immunoglobulin gene, a vector for loxp insertion at both ends of each variable region is constructed. In this case, the inserted loxp is used in the recombination between two chromosomes (a mouse chromosome on which a mouse immunoglobulin gene is located and a human chromosome on which a human immunoglobulin gene is located).

Two vectors (a first vector and a second vector) for loxp insertion at both ends of the variable region of the mouse immunoglobulin gene are constructed.

The first vector includes a first homologous arm used for insertion at the 5'-end of the variable region of the mouse immunoglobulin heavy locus, piggyBac terminal repeat (PB-TR), a promoter, loxm2/66 (first RRS), a blasticidin-resistant gene, a promoter, FRT, and a second homologous arm used for insertion at the 5'-end of the variable region of the mouse immunoglobulin heavy locus.

The second vector includes a third homologous arm used for insertion at the 3'-end of the variable region of the mouse immunoglobulin heavy locus, an inverted zeocin-resistant gene, FRT, lox71 (second RRS), a promoter, a neomycin-resistant gene (NeoR), piggyBac terminal repeat (PB-TR), and a fourth homologous arm used for insertion at the 3'-end of the variable region of the mouse immunoglobulin heavy locus.

In addition, two vectors (a third vector and a fourth vector) for loxp insertion at both ends of the variable region of the human immunoglobulin gene are constructed.

The third vector includes a fifth homologous arm used for insertion at the 5'-end of the variable region of the human immunoglobulin heavy locus, a promoter, a blasticidin-resistant gene, lox66 (third RRS), a promoter, FRT, piggyBac terminal repeat (PB-TR), and a sixth homologous arm used for insertion at the 5'-end of the variable region of the human immunoglobulin heavy locus.

The fourth vector includes a seventh homologous arm used for insertion at the 3'-end of the variable region of the human immunoglobulin heavy locus, piggyBac terminal repeat (PB-TR), an inverted zeocin-resistant gene, FRT, an inverted puroΔTK gene, loxm2/71 (fourth RRS), a promoter, a neomycin-resistant gene (NeoR), and an eighth homologous arm used for insertion at the 3'-end of the variable region of the human immunoglobulin heavy locus.

In this case, the vector construction may vary depending on the insertion location and the type of loxp, and design changes are possible to further include various factors for a selection process.

### 2. Production of Loxp-inserted Cells

Using the vectors, loxp-inserted mouse cells and human cells derived from an individual who is Negro are produced. In this case, when several vectors are used, the introduction of the vectors may be performed sequentially or randomly, and may be performed simultaneously. The selection process of cells into which the vectors are introduced may vary depending on inserted elements.

### 2-1. Production of Loxp-inserted Mouse Cells

The used mouse embryonic stem cells (mESCs) used a basic culture medium, i.e., a 2i medium, wherein the culture medium is a basic culture medium (2i medium) in which an FBS-free N2B27 medium is supplemented with MEK inhibitor PD0325901 (1 µM) and GSK3 inhibitor CHIR99021 (3 µM) (both from Sigma Aldrich, St. Louis, MO, USA), and 1,000 U/ml LIF (Millipore, Billerica, MA, USA), and the mESCs are incubated in an incubator maintained at 5% CO₂, 95% humidity, and 37 °C for proliferation and maintenance. Transient transfection is performed using a Lipofectamine 3000 or Nepa21 (NEPAGENE Co., Ltd., Chiba, Japan) electroporator. For the transient transfection, 1 × 10⁶ cells are prepared in a medium excluding FBS and an antibiotic. 10 µg of the first vector is added thereto, and then the Lipofectamine 3000 reagent is mixed therewith and the resultant mixture is allowed to stand at room temperature for 5 minutes, followed by transfection. Alternatively, 10 µg of the first vector is added thereto, and transfection is performed at 125 V, 5 ms, and 2 pulse. The tranfected mESCs are incubated in an incubator maintained at 5% CO₂, 95% humidity, and 37 °C, for 24 hours to 48 hours.

In order to confirm whether the first vector is inserted at the 5'-end of the variable region of the mouse immunoglobulin heavy locus, the transfected mESCs are treated with blasticidin to confirm the insertion of the first vector through whether the cells survive or not. After blasticidin treatment, viable mESCs are obtained, and the obtained mESCs are transfected with the second vector using the same method as that used for the first vector. The transfected mESCs are incubated for 24 hours to 48 hours in an incubator maintained at 5% CO₂, 95% humidity, and 37 °C. To confirm whether the second vector is inserted at the 3'-end of the variable region of the mouse immunoglobulin heavy locus, the transfected mESCs are treated with G418 (Life Technologies, NY, USA) to confirm the insertion of the second vector through whether mESCs survive or not. After the G418 treatment, viable mESCs are obtained.

To confirm whether the first vector and the second vector are inserted into the same chromosome (a chromosome having a mouse immunoglobulin heavy locus), the mESCs obtained after the G418 treatment are treated with flippase (FLP), which is a recombinase. When the first vector and the second vector are inserted into the same chromosome, recombination is induced by the FRT present in the two vectors and the treated FLP, and thus the variable region of the mouse immunoglobulin heavy locus is inverted. To confirm this, cells in which FRT-FLP recombination has been induced are treated with zeocin, and the insertion of the first vector and the second vector into the same chromosome is confirmed through whether the mESCs survive or not. After the zeocin treatment, surviving mESCs are obtained. The obtained mESCs are mESCs containing a chromosome where loxm2/66 (first RRS) and lox71 (second RRS) are respectively inserted at both ends of the variable region of the mouse immunoglobulin heavy locus. This selection process is for excluding the case where only one of the first vector and the second vector is inserted into the two alleles, since somatic cells generally have two alleles.

### 2-2. Production of Loxp-inserted Human Cells

WS1 cells (ATCC^{®} CRL-1502^{™}, ethnicity is black), which are used human fibroblasts, are incubated in Eagle's Minimum Essential Medium (EMEM; ATCC^{®} 30-2003^{™}) containing 10% fetal bovine serum (FBS) (Corning, Mannasas, VA, USA) and 1% penicillin-streptomycin (Corning, Mannasas, VA, USA) and an incubator maintained at 5% CO₂, 95% humidity, and 37 °C for proliferation and maintenance. Transient transfection is performed using a Lipofectamine 3000 (Invitrogen, Carlsbad, CA, USA) or Nepa21 (NEPAGENE Co., Ltd., Chiba, Japan) electroporator. For the transient transfection, 1 × 10⁶ cells are prepared in a medium excluding FBS and an antibiotic. 10 µg of the third vector is added thereto, and then the Lipofectamin 3000 reagent is mixed therewith and the resultant mixture is allowed to stand at room temperature for 5 minutes, followed by transfection. Alternatively, 10 µg of the third vector is added thereto, and transfection is performed at 150 V, 7.5 ms, and 2 pulses. The transfected WS1 cells are incubated in an incubator maintained at 5% CO₂, 95% humidity, and 37 °C, for 24 hours to 48 hours.

In order to confirm whether the third vector is inserted at the 5'-end of the variable region of the human immunoglobulin heavy locus, the transfected WS1 cells are treated with blasticidin to confirm the insertion of the third vector through whether the WS1 cells survive or not. After blasticidin treatment, viable WS1 cells are obtained, and the obtained WS1 cells are transfected with the foruth vector using the same method as that used for the third vector. The transfected cells are incubated for 24 hours to 48 hours in an incubator maintained at 5% CO₂, 95% humidity, and 37 °C. To confirm whether the foruth vector is inserted at the 3'-end of the variable region of the human immunoglobulin heavy locus, the transfected WS1 cells are treated with G418 (Life Technologies, NY, USA) to confirm the insertion of the fourth vector through whether WS1 cells survive or not. After the G418 treatment, viable WS1 cells are obtained.

To confirm whether the third vector and the fourth vector are inserted into the same chromosome (a chromosome having a human immunoglobulin heavy locus), the WS1 cells obtained after the G418 treatment are treated with flippase (FLP), which is a recombinase. When the third vector and the fourth vector are inserted into the same chromosome, recombination is induced by the FRT present in the two vectors and the treated FLP, and thus the variable region of the human immunoglobulin heavy locus is inverted. To confirm this, WS1 cells in which FRT-FLP recombination has been induced are treated with zeocin, and the insertion of the third vector and the fourth vector into the same chromosome is confirmed through whether the WS1 cells survive or not. After the zeocin treatment, survived WS1 cells are obtained. The obtained WS1 cells are WS1 cells containing a chromosome where lox66 (third RRS) and loxm2/71 (fourth RRS) are respectively inserted at both ends of the variable region of the human immunoglobulin heavy locus. This selection process is for excluding the case where only one of the third vector and the fourth vector is inserted into the two alleles, since somatic cells generally have two alleles.

### 3. Production of Microcells using Loxp-Inserted WS1 Cells

The formation of micronuclei proceeds using colcemid (Life Technologies, Grand island, NY, USA). The selected WS1 cells (cells including a chromosome where lox66 (third RRS) and loxm2/71 (fourth RRS) are respectively inserted at both ends of the variable region of the human immunoglobulin heavy locus) were cultured at a concentration of 1 × 10⁶ cells, and next day, the medium is replaced with EMEM containing 20% FBS, followed by treatment with 0.1 µg/ml of colcemid and incubation for 48 hours in an incubator maintained at 5% CO₂, 95% humidity, and 37 °C. Micronucleation-induced WS1 cells are detached using tryLE (Life Technologies, Grand island, NY, USA), and then washed with serum-free EMEM, followed by centrifugation (LABOGENE CO., Ltd, KOREA) at 1,000 rpm for 5 minutes. After the centrifugation, the cells are suspended in pre-warmed serum-free EMEM: Percoll ((Sigma Aldrich, St. Louis, MO, USA) (1:1 (v:v)), and treated with cytochalsin B (Sigma Aldrich, St. Louis, MO, USA) to a final concentration of 10 µg/ml. The cells are centrifuged (LABOGENE) at 16000 g and 34 °C to 37 °C for 1 hour to 1.5 hours to separate whole cells and microcells. The separated whole cells and microcells are transferred to a 50 ml tube, and serum-free EMEM is added thereto, followed by centrifugation at 500 g for 10 minutes. The supernatant is carefully removed, 10 ml of serum-free EMEM is added to a pellet attached to the surface of the tube, and microcells having a size of 8 µm or less are separated using an 8 µm filter (GE Healthcare, CHICAGO, IL, USA). A supernatant including the separated microcells is centrifuged again at 400 g for 10 minutes. For the centrifuged microcells, a 5 µm filter is used using the same method as that used for an 8 µm filter. The finally separated microcells are counted through a Nicon eclipse TS100 optical microscope (Nicon Instruments, Melville, NY, USA). Through this, microcells are obtained from WS1 cells.

### 4. Production of Fusion Cells using Microcells and Loxp-inserted Mouse Cells

The separated human microcells and loxp-inserted mESCs to be used as recipient cells (cells including a chromosome where loxm2/66 (first RRS) and lox71 (second RRS) are respectively inserted at both ends of the variable region of a mouse immunoglobulin heavy locus) are prepared. The mESCs are treated with TryLE and centrifuged. The centrifuged mESCs are washed with 1xDPBS and the number of cells is calculated using a hemacytometer. The human microcells and the mESCs are fused using a HVJ-E protein (Cosmo Bio Co., Ltd., Tokyo, Japan) according to a suspension method. The human microcells and the mESCs are used at a ratio of 1:4. The prepared human microcells and mESCs are each washed using 500 µl of 1x cell fusion buffer, which is cold. The human microcells and mESCs contained in the buffer are centrifuged at 300 g and 4 °C for 5 minutes. 25 µl of 1x cell fusion buffer per 2 × 10⁵ cells is added to the mESCs, and the same volume of 1x cell fusion buffer is also added to the human microcells. The mESCs and the human microcells are mixed, 5-10 µl of the HVJ-E protein is added thereto, and then the cells are left on ice for 5 minutes. The mixture is allowed to stand in a 37 °C water bath for 15 minutes. At this time, tapping is performed every 5 minutes. After cell fusion between the human microcells and the mESCs is completed, centrifugation is performed at 300 g for 5 minutes to remove remaining HVJ-E protion. The fusion cells are incubated for 48 hours in the culture medium of the mESCs and an incubator maintained at 5% CO₂, 95% humidity, and 37 °C. The produced fusion cells are mESCs containing a human chromosome (a chromosome containing the variable region of a human immunoglobulin heavy locus, at which lox66 (third RRS) and loxm2/71 (fourth RRS) are inserted) and a mouse chromosome (a chromosome containing the variable region of a mouse immunoglobulin heavy locus, at which loxm2/66 (first RRS) and lox71 (second RRS) are inserted).

### 5. Production of Cells Having Negro-Derived Humanized Immunoglobulin Gene by Chromosome Exchange

The produced fusion cells are treated with a recombinase to induce substitution (or replacement) between the variable region of a mouse immunoglobulin heavy locus and the variable region of a human immunoglobulin heavy locus, thereby producing fusion cells having a humanized immunoglobulin heavy locus, i.e., mESCs. In this case, the humanized immunoglobulin heavy locus is a locus containing a variable region derived from a human immunoglobulin heavy chain gene and the constant region of a mouse immunoglobulin heavy chain gene.

100 µl of an opti-MEM medium excluding FBS and an antibiotic is added to 1 × 10⁶ fusion cells (mESCs). 10 µg of a pCMV-Cre (System Biosciences, LLC, Palo Alto, CA, USA) vector is added thereto, and transfection is performed at 125 V, 5 ms, and 2 pulses. 300 µl of a 2i medium is added and mixed well with the fusion cells, followed by transfer to a 100-mm dish, and the cells are incubated for 48 hours in an incubator maintained at 5% CO₂, 95% humidity, and 37 °C.

To confirm whether a chromosome having a humanized immunoglobulin locus is produced in the fusion cells treated with Cre recombinase, the fusion cells treated with Cre recombinase are treated with antibiotics (puromycin, G418, and neomycin). In the Cre recombinase-treated fusion cells, recombination is induced, by the Cre recombinase, between the human chromosome (a chromosome containing the variable region of a human immunoglobulin heavy locus, at which lox66 (third RRS) and loxm2/71 (fourth RRS) are inserted) and the mouse chromosome (a chromosome containing the variable region of a mouse immunoglobulin heavy locus, at which loxm2/66 (first RRS) and lox71 (second RRS) are inserted). The first RRS in the mouse chromosome is paired with the fourth RRS in the human chromosome, and the second RRS in the mouse chromosome is paired with the third RRS in the human chromosome. Cre recombinase induces recombination by recognizing the pairing of the RRSs.

As a result, a first recombinant chromosome (mouse chromosome having a humanized immunoglobulin locus), in which the variable region of the mouse immunoglobulin heavy locus of the mouse chromosome is substituted with the variable region of a human immunoglobulin heavy chain gene), and a second recombinant chromosome, in which the variable region of the human immunoglobulin heavy locus of the human chromosome is substituted with the variable region of a mouse immunoglobulin heavy chain gene, are produced. The first recombinant chromosome is a chromosome in which the remaing region excluding the variable region of the human immunoglobulin heavy chain gene has a mouse gene (e.g., the constant region of the mouse immunoglobulin heavy locus is a mouse gene). The second recombinant chromosome is a chromosome in which the remaining region excluding the variable region of the mouse immunoglobulin heavy chain gene has a human gene (e.g., the constant region of the human immunoglobulin heavy locuse is a human gene).

After the treatment with antibiotics (puromycin, G418, and neomycin), surviving mESCs are obtained. The obtained cells are mESCs containing the first recombinant chromosome and the second recombinant chromosome.

The obtained mESCs containing the first recombinant chromosome and the second recombinant chromosome are treated with piggyBac transposase to remove RRS, a puroΔTK gene, a neomycin-resistant gene (NeoR), a zeocin-resistant gene, and FRT, which are included in the first recombinant chromosome and the second recombinant chromosome. At this time, cells including the recombinant chromosome, from which RRS, a puroΔTK gene, a neomycin-resistant gene (NeoR), a zeocin-resistant gene, and FRT have been removed, are selected by fialuridine (FIAU) treatment.

The recombinant chromosome may vary depending on the location, direction, and pairing of RRS. A desired recombinant chromosome can be produced by changing the design of a vector as described above.

### 6. Production of Homozygous Transgenic Mouse using mESCs having Negro-Derived Humanized Immunoglobulin Locus

A homozygous transgenic mouse is produced using mESCs having a Negro-derived humanized immunoglobulin locus.

The obtained mESCs having a humanized immunoglobulin locus are implanted into a blastula through blastocyst injection to produce a chimeric blastocyst. The produced chimeric blastocyst is implanted into the uterus of a surrogate mother to give birth to each living mouse offspring. The obtained living mouse offsprings are chimeric transgenic mice, and the chimeric transgenic mice are crossed to produce a homozygous transgenic mouse. In the produced homozygous transgenic mouse, the variable region of an immunoglobulin heavy locus on the genome is humanized (substituted (or replaced) with the variable region of a Negro-derived humanized immunoglobulin gene. In this case, the homozygous transgenic mouse can be produced using various methods other than blastocyst injection.

### Example 1-2. Method of Producing Homozygous Transgenic Mouse having Mongoloid Race-Derived Humanized Immunoglobulin Gene

The present example relates to the production of a humanized homozygous transgenic mouse by substitution (or replacement) of the variable region of a mouse immunoglobulin heavy locus with the variable region of a human immunoglobulin gene derived from an individual belonging to the Mongoloid race. In the corresponding example, a homozygous transgenic mouse having a Mongoloid race-derived humanized immunoglobulin gene is produced using a chromosome exchange technique (e.g., AiCE technique (see PCT/KR2019/015351)).

The corresponding method is the same as that described above in "Example 1-1. Method of Producing Homozygous Transgenic Mouse having Humanized Immunoglobulin Gene derived from Negro," and human cells used in the experiment are OUMS-36 cells (JCRB1006.0, the race is Japanese), which are human fibroblasts derived from an individual belonging to the Mongoloid race. The OUMS-36 cells are incubated in Dulbecco's Modified Eagle's Medium (DMEM; Corning, Mannasas, VA, USA) containing 10% fetal bovine serum (FBS; Corning, Mannasas, VA, USA) and 1% penicillin-streptomycin (Corning, Mannasas, VA, USA) and an incubator maintained at 5% CO₂, 95% humidity, and 37 °C for proliferation and maintenance.

### Example 1-3. Method of Producing Heterozygous Transgenic Mouse having Negro-Derived Humanized Immunoglobulin Gene and Mongoloid Race-Derived Humanized Immunoglobulin Gene

The present example relates to the production of a heterozygous transgenic mouse having a Negro-derived humanized immunoglobulin gene and a Mongoloid race-derived humanized immunoglobulin gene.

The heterozygous transgenic mouse is produced by crossing the produced homozygous transgenic mouse having a Negro-derived humanized immunoglobulin gene and the produced homozygous transgenic mouse having a Mongoloid race-derived humanized immunoglobulin gene. The produced heterozygous transgenic mouse is a transgenic mouse having hetero-alleles of a humanized immunoglobulin locus, i.e., a Negro-derived humanized immunoglobulin gene and a Mongoloid race-derived humanized immunoglobulin gene.

### Example 1-4. Method of Producing Homozygous Transgenic Mouse having Caucasian-Derived Humanized Immunoglobulin Gene

The present example relates to the production of a humanized homozygous transgenic mouse by substitution (or replacement) of the variable region of a mouse immunoglobulin heavy locus with the variable region of a human immunoglobulin gene derived from an individual, who is Caucasian. In the corresponding example, a homozygous transgenic mouse having a Caucasian-derived humanized immunoglobulin gene is produced using a chromosome exchange technique (e.g., AiCE technique (see PCT/KR2019/015351)).

The corresponding method is the same as that described above in "Example 1-1. Method of Producing Homozygous Transgenic Mouse having Humanized Immunoglobulin Gene derived from Negro," and human cells used in the experiment are BJ cells (ATCC^{®} CRL-2522^{™}, ethnicity is white), which are human fibroblasts derived from an individual belonging to the Caucasoid race. The BJ cells are incubated in Eagle's Minimum Essential Medium (EMEM) (ATCC^{®} 30-2003^{™}) containing 10% fetal bovine serum (FBS; Corning, Mannasas, VA, USA) and 1% penicillin-streptomycin (Corning, Mannasas, VA, USA) and an incubator maintained at 5% CO₂, 95% humidity, and 37 °C for proliferation and maintenance.

### Example 1-5. Method of Producing Heterozygous Transgenic Mouse having Negro-Derived Humanized Immunoglobulin Gene and Caucasian-Derived Humanized Immunoglobulin Gene

The present example relates to the production of a heterozygous transgenic mouse having a Negro-derived humanized immunoglobulin gene and a Caucasian-derived humanized immunoglobulin gene.

The heterozygous transgenic mouse is produced by crossing the produced homozygous transgenic mouse having a Negro-derived humanized immunoglobulin gene and the produced homozygous transgenic mouse having a Caucasian-derived humanized immunoglobulin gene. The produced heterozygous transgenic mouse is a transgenic mouse having hetero-alleles of a humanized immunoglobulin locus, i.e., a Negro-derived humanized immunoglobulin gene and a Caucasian-derived humanized immunoglobulin gene.

### Example 1-6. Method of Producing Heterozygous Transgenic Mouse having Mongoloid Race-Derived Humanized Immunoglobulin Gene and Caucasian-Derived Humanized Immunoglobulin Gene

The present example relates to the production of a heterozygous transgenic mouse having a Mongoloid race-derived humanized immunoglobulin gene and a Caucasian-derived humanized immunoglobulin gene.

The heterozygous transgenic mouse is produced by crossing the produced homozygous transgenic mouse having a Mongoloid race-derived humanized immunoglobulin gene and the produced homozygous transgenic mouse having a Caucasian-derived humanized immunoglobulin gene. The produced heterozygous transgenic mouse is a transgenic mouse having hetero-alleles of a humanized immunoglobulin locus, i.e., a Mongoloid race-derived humanized immunoglobulin gene and a Caucasian-derived humanized immunoglobulin gene.

In addition to the heterozygous transgenic animals described in the examples, a heterozygous transgenic mouse having humanized immunoglobulin genes derived from different races may be produced based on the examples.

A homozygous transgenic mouse having humanized immunoglobulin genes derived from various races may be produced using the method described above in "Example 1-1. Method of Producing Homozygous Transgenic Mouse having Humanized Immunoglobulin Gene derived from Negro." This may vary depending on human cells used in the experiments. The human cells used in the experiments may be haman cells derived from Caucasian (the Caucasoid race), Mongolian (the Mongoloid race), Negro (Negroid), the Malay race, Polynesian, or the Australoid race, and by using this, a homozygous transgenic mouse having a humanized immunoglobulin gene derived from Caucasian (the Caucasoid race), Mongolian (the Mongoloid race), Negro (Negroid), the Malay race, Polynesian, or the Australoid race may be produced.

A heterozygous transgenic mouse is produced by crossing the produced homozygous transgenic mice having humanized immunoglobulin genes derived from different races. The produced mouse may be a heterozygous transgenic mouse derived from Caucasian and the Mongoloid race (a heterozygous transgenic mouse having a Caucasian-derived humanized immunoglobulin gene and a Mongoloid race-derived humanized immunoglobulin gene), a heterozygous transgenic mouse derived from Caucasian and Negro, a heterozygous transgenic mouse derived from Caucasian and the Malay race, a heterozygous transgenic mouse derived from Caucasian and Polynesian, a heterozygous transgenic mouse derived from the Mongoloid race and Negro, a heterozygous transgenic mouse derived from the Mongoloid race and the Malay race, a heterozygous transgenic mouse derived from the Mongoloid race and Polynesian, a heterozygous transgenic mouse derived from Negro and the Malay race, a heterozygous transgenic mouse derived from Negro and Polynesian, or a heterozygous transgenic mouse derived from the Malay race and Polynesian. In addition, even in the case of using human cells derived from different races other than the above-described races, a heterozygous transgenic mouse may be produced using the same method.

## Claims

1. A heterozygous transgenic non-human animal comprising humanized immunoglobulin loci,
wherein the humanized immunoglobulin loci comprise two alleles of humanized immunoglobulin gene,
wherein the humanized immunoglobulin gene includes an unrearranged variable region of human immunoglobulin gene and a constant region of non-human animal immunoglobulin gene,
wherein the two alleles of humanized immunoglobulin gene are hetero-alleles and comprise a first humanized immunoglobulin gene and a second humanized immunoglobulin gene,
wherein the first humanized immunoglobulin gene includes a first unrearranged variable region of a first human immunoglobulin gene, the second humanized immunoglobulin gene includes a second unrearranged variable region of a second human immunoglobulin gene, in which the first unrearranged variable region and the second unrearranged variable region are hetero variable regions,
wherein the first human immunoglobulin gene is derived from an individual belonging to a first population, the second human immunoglobulin gene is derived from an individual belonging to a second population, in which the first population and the second population are different and are selected from the group consisting of Caucasian (Caucasoid race), Mongolian (Mongoloid race), Negro (Negroid), Malay and Polynesian, Australoid race.

2. The heterozygous transgenic non-human animal of claim 1,
wherein the humanized immunoglobulin gene is a humanized immunoglobulin heavy gene.

3. The heterozygous transgenic non-human animal of claim 2,
wherein the unrearranged variable region comprises one or more V segments, one or more D segments and one or more J segments.

4. The heterozygous transgenic non-human animal of claim 1,
wherein the humanized immunoglobulin gene is a humanized immunoglobulin kappa gene or humanized immunoglobulin lambda gene.

5. The heterozygous transgenic non-human animal of claim 4,
wherein the unrearranged variable region comprises one or more V segments and one or more J segments.

6. The heterozygous transgenic non-human animal of claim 1,
wherein the hetero variable regions have one or more of the following differences:
i) synonymous mutation or nonsynonymous mutation by Single nucleotide polymorphism (SNP);
ii) allelic variation of a V segment;
iii) copy number variation (CNV) of a segment;
iv) copy number variation (CNV) of an open reading frame (ORF); and
v) deletion, insertion or duplication of nucleic acids having a length of 8-75 kb.

7. The heterozygous transgenic non-human animal of claim 1,
wherein the non-human animal is an avian or a mammal other than human.

8. The heterozygous transgenic non-human animal of claim 7,
wherein the mammal other than human is a rodent, an ungulate or a non-human primate.

9. The heterozygous transgenic non-human animal of claim 7,
wherein the non-human animal is a mouse, rat, rabbit, goat, cow, pig, camel, chimpanzee, monkey, chicken or quail.

10. A heterozygous transgenic non-human animal comprising humanized immunoglobulin loci,
wherein the humanized immunoglobulin loci comprise two alleles of humanized immunoglobulin gene,
wherein the humanized immunoglobulin gene includes an unrearranged variable region of human immunoglobulin gene and a constant region of non-human animal immunoglobulin gene,
wherein the two alleles of humanized immunoglobulin gene are hetero-alleles and comprise a first humanized immunoglobulin gene and a second humanized immunoglobulin gene,
wherein the first humanized immunoglobulin gene includes a first unrearranged variable region of a first human immunoglobulin gene, the second humanized immunoglobulin gene includes a second unrearranged variable region of a second human immunoglobulin gene, in which the first unrearranged variable region and the second unrearranged variable region are hetero variable regions,
wherein the first human immunoglobulin gene is derived from a first individual, the second human immunoglobulin gene is derived from a second individual, in which the first individual and the second individual are different.

11. The heterozygous transgenic non-human animal of claim 10,
wherein the humanized immunoglobulin gene is a humanized immunoglobulin heavy gene.

12. The heterozygous transgenic non-human animal of claim 11,
wherein the unrearranged variable region comprises one or more V segments, one or more D segments and one or more J segments.

13. The heterozygous transgenic non-human animal of claim 10,
wherein the humanized immunoglobulin gene is a humanized immunoglobulin kappa gene or humanized immunoglobulin lambda gene.

14. The heterozygous transgenic non-human animal of claim 13,
wherein the unrearranged variable region comprises one or more V segments and one or more J segments.

15. The heterozygous transgenic non-human animal of claim 10,
wherein the hetero variable regions have one or more of the following differences:
i) synonymous mutation or nonsynonymous mutation by Single nucleotide polymorphism (SNP);
ii) allelic variation of a V segment;
iii) copy number variation (CNV) of a segment;
iv) copy number variation (CNV) of an open reading frame (ORF); and
v) deletion, insertion or duplication of nucleic acids having a length of 8-75kb.

16. The heterozygous transgenic non-human animal of claim 10,
wherein the non-human animal is an avian or a mammal other than human.

17. The heterozygous transgenic non-human animal of claim 16,
wherein the mammal other than human is a rodent, an ungulate or a non-human primate.

18. The heterozygous transgenic non-human animal of claim 16,
wherein the non-human animal is a mouse, rat, rabbit, goat, cow, pig, camel, chimpanzee, monkey, chicken or quail.
